(19) **Europäisches Patentamt / European Patent Office / Office européen des brevets**

(11) **EP 1 449 922 B1**

(12) **EUROPEAN PATENT SPECIFICATION**

(45) Date of publication and mention
of the grant of the patent:
**15.08.2007 Bulletin 2007/33**

(51) Int Cl.:
*C12N 15/31* [(2006.01)]    *A61K 39/04* [(2006.01)]
*C07K 14/35* [(2006.01)]    *C12N 15/62* [(2006.01)]
*A61K 38/16* [(2006.01)]    *G01N 33/569* [(2006.01)]
*C12Q 1/68* [(2006.01)]    *C07K 16/12* [(2006.01)]

(21) Application number: **04076605.7**

(22) Date of filing: **01.04.1998**

(54) **Nucleic acid fragments and polypeptide fragments derived from M. tuberculosis**

Nukleinsäure- und Polypeptidfragmente von M. tuberculosis

Fragments d'acide nucléique et fragments polypeptidiques derivés de M. tuberculosis

(84) Designated Contracting States:
**AT BE CH CY DE DK ES FI FR GB GR IE IT LI LU MC NL PT SE**

(30) Priority: **02.04.1997 DK 37697**
**18.04.1997 US 44624 P**
**10.11.1997 DK 127797**
**05.01.1998 US 70488 P**

(43) Date of publication of application:
**25.08.2004 Bulletin 2004/35**

(62) Document number(s) of the earlier application(s) in accordance with Art. 76 EPC:
**98913536.3 / 0 972 045**

(73) Proprietor: **Statens Serum Institut**
**2300 Copenhagen S (DK)**

(72) Inventors:
 • **Andersen, Peter**
 **2700 Brönshöj (DK)**
 • **Florio, Walter**
 **Carrara (MS) (IT)**

• **Oettinger, Thomas**
 **2900 Hellerup (DK)**
• **Rasmussen, Peter Birk**
 **2000 Frederiksberg (DK)**
• **Rosenkrands, Ida**
 **3500 Vaerlöse (DK)**
• **Skjöt, Rikke Louise Vinther**
 **2640 Hedehusene (DK)**
• **Weldingh, Karin**
 **3500 Vaerlöse (DK)**

(74) Representative: **Plougmann & Vingtoft A/S**
**Sundkrogsgade 9,**
**P.O. Box 831**
**2100 Copenhagen Ø (DK)**

(56) References cited:
**WO-A-97/09428**      **WO-A-97/09429**

• **DATABASE EMBL EBI; 30 May 2000 (2000-05-30), NIELSEN R V: "ESAT-6 like protein eshx (10 kDa antigen CFP7)" XP002296455 Database accession no. O53693**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

FIELD OF THE INVENTION

The present invention relates to a number of immunologically active, novel polypeptide fragments derived from the CFP7 antigen of the *Mycobacterium tuberculosis,* vaccines and other immunologic compositions containing the fragments as immunogenic components, and methods of production and use of the polypeptides. The invention also relates to novel nucleic acid fragments derived from the CFP7 antigen of the *M. tuberculosis* which are useful in the preparation of the polypeptide fragments of the invention or in the diagnosis of infection with *M. tuberculosis.*

BACKGROUND OF THE INVENTION

Human tuberculosis (hereinafter designated "TB") caused by *Mycobacterium tuberculosis* is a severe global health problem responsible for approximately 3 million deaths annually, according to the WHO. The worldwide incidence of new TB cases has been progressively falling for the last decade but the recent years has markedly changed this trend due to the advent of AIDS and the appearance of multidrug resistant strains of *M. tuberculosis.*

The only vaccine presently available for clinical use is BCG, a vaccine which efficacy remains a matter of controversy. BCG generally induces a high level of acquired resistance in animal models of TB, but several human trials in developing countries have failed to demonstrate significant protection. Notably, BCG is not approved by the FDA for use in the United States.

This makes the development of a new and improved vaccine against TB an urgent matter which has been given a very high priority by the WHO. Many attempts to define protective mycobacterial substances have been made, and from 1950 to 1970 several investigators reported an increased resistance after experimental vaccination. However, the demonstration of a specific long-term protective immune response with the potency of BCG has not yet been achieved by administration of soluble proteins or cell wall fragments, although progress is currently being made by relying on polypeptides derived from short term-culture filtrate, cf. the discussion below.

Immunity to *M. tuberculosis* is characterized by three basic features; i) Living bacilli efficiently induces a protective immune response in contrast to killed preparations; ii) Specifically sensitized T lymphocytes mediate this protection; iii) The most important mediator molecule seems to be interferon gamma (INF-γ).

Short term-culture filtrate (ST-CF) is a complex mixture of proteins released from *M. tuberculosis* during the first few days of growth in a liquid medium (Andersen *et al.,* 1991). Culture filtrates has been suggested to hold protective antigens recognized by the host in the first phase of TB infection (Andersen *et al.* 1991, Orme *et al.* 1993). Recent data from several laboratories have demonstrated that experimental subunit vaccines based on culture filtrate antigens can provide high levels of acquired resistance to TB (Pal and Horwitz, 1992; Roberts *et al.,* 1995; Andersen, 1994; Lindblad *et al.,* 1997). Culture filtrates are, however, complex protein mixtures and until now very limited information has been available on the molecules responsible for this protective immune response. In this regard, only two culture filtrate antigens have been described as involved in protective immunity, the low mass antigen ESAT-6 (Andersen *et al.,* 1995 and EP-A-0 706 571) and the 31 kDa molecule Ag85B (EP-0 432 203).

There is therefore a need for the identification of further antigens involved in the induction of protective immunity against TB in order to eventually produce an effective subunit vaccine.

WO97709428 (Corixa Corp.) 13 March 1997 and WO97/09429 (Corixa Corp.) 13 March 1997 both disclose nucleic acid sequences and their corresponding polypeptides, inter alia ESAT6, derived from Mycobacterium tuberculosis and their use for immunization and diagnosis of M tuberculosis infection. The polypeptides of the present invention have an overall sequence identity of 29.2% with the polypeptide ESAT6. The highest identity, namely 71.4% was found in an overlap of 7 amino acids.

OBJECT OF THE INVENTION

It is an object of the invention to provide novel antigens which are effective as components in a subunit vaccine against TB or which are useful as components in diagnostic compositions for the detection of infection with mycobacteria, especially virulence-associated mycobacteria. The novel antigens may also be important drug targets.

SUMMARY OF THE INVENTION

The present invention is i.a. based on the identification and characterization of a number of previously uncharacterized culture filtrate antigens from *M. tuberculosis.* In animal models of TB, T cells mediating immunity are focused predominantly to antigens in the regions 6-12 and 17-30 kDa of STCF. In the present invention an antigen in the low molecular weight region (CFP7) has been identified.

The encoding gene for the antigen has been determined, the distribution of the antigen in various mycobacterial strains investigated and the biological activity of the product characterized. The antigene has potential for vaccine purposes as well as for diagnostic purposes, since the antigen is secreted by metabolizing mycobacteria.

The following table lists the antigen of the invention by the name used herein as well as by reference to relevant SEQ ID NOs of full amino acid sequence and sequence of DNA encoding the antigen:

| Antigen | N-terminal sequence SEQ ID NO: | Nucleotide sequence SEQ ID NO: | Amino acid sequence SEQ ID NO: |
|---------|-------------------------------|-------------------------------|-------------------------------|
| CFP7 | | 1 | 2 |

It is well-known in the art that T-cell epitopes are responsible for the elicitation of the acquired immunity against TB, whereas B-cell epitopes are without any significant influence on acquired immunity and recognition of mycobacteria *in vivo*. Since such T-cell epitopes are linear and are known to have a minimum length of 6 amino acid residues, the present invention is especially concerned with the identification and utilisation of such T-cell epitopes.

Hence, in its broadest aspect the invention relates to a substantially pure polypeptide fragment which

a) comprises an amino acid sequence as shown in SEQ ID NO: 2,

b) comprises a subsequence of the polypeptide fragment defined in a) which has a length of at least 12 amino acid residues, said subsequence being immunologically equivalent to the polypeptide defined in a) with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex, or

c) comprises an amino acid sequence having a sequence identity with the polypeptide defined in a) or the subsequence defined in b) of at least 80% and at the same time being immunologically equivalent to the polypeptide defined in a) with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex,

said preparation containing at most 5% by weight of other polypeptide material with which the polypeptide fragment is natively associated, with the proviso that, when consisting of the amino acid sequence 1-96 of SEQ ID NO: 2, the polypeptide fragment is free of any other antigen from bacteria belonging to the tuberculosis complex.

Other parts of the invention pertains to the DNA fragments encoding a polypeptide with the above definition as well as to DNA fragments useful for determining the presence of DNA encoding such polypeptides.

DETAILED DISCLOSURE OF THE INVENTION

In the present specification and claims, the term "polypeptide fragment" denotes both short peptides with a length of at least two amino acid residues and at most 10 amino acid residues, oligopeptides (11-100 amino acid residues), and longer peptides (the usual interpretation of "polypeptide", *i.e.* more than 100 amino acid residues in length) as well as proteins (the functional entity comprising at least one peptide, oligopeptide, or polypeptide which may be chemically modified by being glycosylated, by being lipidated, or by comprising prosthetic groups). The definition of polypeptides also comprises native forms of peptides/proteins in mycobacteria as well as recombinant proteins or peptides in any type of expression vectors transforming any kind of host, and also chemically synthesized peptides.

In the present context the term "substantially pure polypeptide fragment" means a polypeptide preparation which contains at most 5% by weight of other polypeptide material with which it is natively associated (lower percentages of other polypeptide material are preferred, e.g. at most 4%, at most 3%, at most 2%, at most 1%, and at most ½%). It is preferred that the substantially pure polypeptide is at least 96% pure, *i.e.* that the polypeptide constitutes at least 96% by weight of total polypeptide material present in the preparation, and higher percentages are preferred, such as at least 97%, at least 98%, at least 99%, at least 99,25%, at least 99,5%, and at least 99,75%. It is especially preferred that the polypeptide fragment is in "essentially pure form", *i.e.* that the polypeptide fragment is essentially free of any other antigen with which it is natively associated, *i.e.* free of any other antigen from bacteria belonging to the tuberculosis complex. This can be accomplished by preparing the polypeptide fragment by means of recombinant methods in a non-mycobacterial host cell as will be described in detail below, or by synthesizing the polypeptide fragment by the well-known methods of solid or liquid phase peptide synthesis, e.g. by the method described by Merrifield or variations thereof.

The term "subsequence" when used in connection with a polypeptide of the invention having SEQ ID NO 2 denotes any continuous stretch of at least 12 amino acid residues taken from the *M. tuberculosis* derived polypeptide in SEQ ID NO: 2 and being immunological equivalent thereto with respect to the ability of conferring increased resistance to infections with bacteria belonging to the tuberculosis complex. Thus, included is also a polypeptide from different sources, such as other bacteria or even from eukaryotic cells.

When referring to an "immunologically equivalent" polypeptide is herein meant that the polypeptide, when formulated in a vaccine or a diagnostic agent (*i.e.* together with a pharmaceutically acceptable carrier or vehicle and optionally an adjuvant), will

I) confer, upon administration (either alone or as an immunologically active constituent together with other antigens), an acquired increased specific resistance in a mouse and/or in a guinea pig and/or in a primate such as a human being against infections with bacteria belonging to the tuberculosis complex which is at least 20% of the acquired increased resistance conferred by *Mycobacterium bovis* BCG and also at least 20% of the acquired increased resistance conferred by the parent polypeptide comprising SEQ ID NO: 2 (said parent polypeptide having substantially the same relative location and pattern in a 2DE gel prepared as the 2DE gel shown in Fig. 6, cf. the examples), the acquired increased resistance being assessed by the observed reduction in mycobacterial counts from spleen, lung or other organ homogenates isolated from the mouse or guinea pig receiving a challenge infection with a virulent strain of *M. tuberculosis*, or, in a primate such as a human being, being assessed by determining the protection against development of clinical tuberculosis in a vaccinated group versus that observed in a control group receiving a placebo or BCG (preferably the increased resistance is higher and corresponds to at least 50% of the protective immune response elicited by *M. bovis* BCG, such as at least 60%, or even more preferred to at least 80% of the protective immune response elicited by *M. bovis* BCG, such as at least 90%; in some cases it is expected that the increased resistance will supersede that conferred by *M. bovis* BCG, and hence it is preferred that the resistance will be at least 100%, such as at least 110% of said increased resistance); and/or

II) elicit a diagnostically significant immune response in a mammal indicating previous or ongoing sensitization with antigens derived from mycobacteria belonging to the tuberculosis complex; this diagnostically significant immune response can be in the form of a delayed type hypersensitivity reaction which can e.g. be determined by a skin test, or can be in the form of IFN-γ release determined e.g. by an IFN-γ assay as described in detail below. A diagnostically significant response in a skin test setup will be a reaction which gives rise to a skin reaction which is at least 5 mm in diameter and which is at least 65% (preferably at least 75% such as at the least 85%) of the skin reaction (assessed as the skin reaction diameter) elicited by the parent polypeptide comprising SEQ ID NO: 2.

The ability of the polypeptide fragment to confer increased immunity may thus be assessed by measuring in an experimental animal, e.g. a mouse or a guinea pig, the reduction in mycobacterial counts from the spleen, lung or other organ homogenates isolated from the experimental animal which have received a challenge infection with a virulent strain of mycobacteria belonging to the tuberculosis complex after previously having been immunized with the polypeptide, as compared to the mycobacterial counts in a control group of experimental animals infected with the same virulent strain, which experimental animals have not previously been immunized against tuberculosis. The comparison of the mycobacterial counts may also be carried out with mycobacterial counts from a group of experimental animals receiving a challenge infection with the same virulent strain after having been immunized with *Mycobacterium bovis* BCG.

The mycobacterial counts in homogenates from the experimental animals immunized with a polypeptide fragment according to the present invention must at the most be 5 times the counts in the mice or guinea pigs immunized with *Mycobacterium bovis* BCG, such as at the most 3 times the counts, and preferably at the most 2 times the counts.

A more relevant assessment of the ability of the polypeptide fragment of the invention to confer increased resistance is to compare the incidence of clinical tuberculosis in two groups of individuals (e.g. humans or other primates) where one group receives a vaccine as described herein which contains an antigen of the invention and the other group receives either a placebo or an other known TB vaccine (e.g. BCG). In such a setup, the antigen of the invention should give rise to a protective immunity which is significantly higher than the one provided by the administration of the placebo (as determined by statistical methods known to the skilled artisan).

The "tuberculosis-complex" has its usual meaning, *i.e.* the complex of mycobacteria causing TB which are *Mycobacterium tuberculosis, Mycobacterium bovis, Mycobacterium bovis* BCG, and *Mycobacterium africanum.*

In the present context the term "metabolizing mycobacteria" means live mycobacteria that are multiplying logarithmically and releasing polypeptides into the culture medium wherein they are cultured.

The term "sequence identity" indicates a quantitative measure of the degree of homology between two amino acid sequences or between two nucleotide sequences of equal length: The sequence identity can be calculated as

$$\frac{(N_{ref} - N_{dif})100}{N_{ref}}$$ , wherein

$N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8) .

The sequence identity is used here to illustrate the degree of identity between the amino acid sequence of a given polypeptide and the amino acid sequence shown in SEQ ID NO: 2. The amino acid sequence to be compared with the amino acid sequence shown in SEQ ID NO: 2 may be deduced from a DNA sequence, e.g. obtained by hybridization as defined below, or may be obtained by conventional amino acid sequencing methods. The sequence identity is preferably determined on the amino acid sequence of a mature polypeptide, *i.e.* without taking any leader sequence into consideration.

As appears from the above disclosure, polypeptides which are not identical to the polypeptide having SEQ ID NO: 2 are embraced by the present invention. The invention allows for minor variations which do not have an adverse effect on immunogenicity compared to the parent sequences and which may give interesting and useful novel binding properties or biological functions and immunogenicities etc.

Each polypeptide fragment may thus be characterized by specific amino acid and nucleic acid sequences. It will be understood that such sequences include analogues and variants produced by recombinant methods wherein such nucleic acid and polypeptide sequences have been modified by substitution, insertion, addition and/or deletion of one or more nucleotides in said nucleic acid sequences to cause the substitution, insertion, addition or deletion of one or more amino acid residues in the recombinant polypeptide. When the term DNA is used in the following, it should be understood that for the number of purposes where DNA can be substituted with RNA, the term DNA should be read to include RNA embodiments which will be apparent for the man skilled in the art. For the purposes of hybridization, PNA may be used instead of DNA, as PNA has been shown to exhibit a very dynamic hybridization profile (PNA is described in Nielsen P E et al., 1991, Science 254: 1497-1500).

In both immunodiagnostics and vaccine preparation, it is often possible and practical to prepare antigens from segments of a known immunogenic protein or polypeptide. Certain epitopic regions may be used to produce responses similar to those produced by the entire antigenic polypeptide. Potential antigenic or immunogenic regions may be identified by any of a number of approaches, e.g., Jameson-Wolf or Kyte-Doolittle antigenicity analyses or Hopp and Woods (1981) hydrophobicity analysis (see, e.g., Jameson and Wolf, 1988; Kyte and Doolittle, 1982; or U.S. Patent No. 4,554,101). Hydrophobicity analysis assigns average hydrophilicity values to each amino acid residue from these values average hydrophilicities can be calculated and regions of greatest hydrophilicity determined. Using one or more of these methods, regions of predicted antigenicity may be derived from the amino acid sequence assigned to the polypeptides of the invention.

Alternatively, in order to identify relevant T-cell epitopes which are recognized during an immune response, it is also possible to use a "brute force" method: Since T-cell epitopes are linear, deletion mutants of the polypeptide having SEQ ID NO: 2 will, if constructed systematically, reveal what regions of the polypeptides are essential in immune recognition, e.g. by subjecting these deletion mutants to the IFN-γ assay described herein. Another method utilises overlapping oligomers (preferably synthetic having a length of e.g. 20 amino acid residues) derived from the polypeptide having SEQ ID NO: 2. Some of these will give a positive response in the IFN-γ assay whereas others will not.

In a preferred embodiment of the invention, the polypeptide fragment of the invention comprises an epitope for a T-helper cell.

Although the minimum length of a T-cell epitope has been shown to be at least 6 amino acids, it is normal that such epitopes are constituted of longer stretches of amino acids. Hence it is preferred that the polypeptide fragment of the invention has a length of amino acid residues.

In another preferred embodiment, the polypeptide fragment of the invention is free from any signal sequence; this is especially interesting when the polypeptide fragment is produced synthetically but even when the polypeptide fragments are produced recombinantly it is normally acceptable that they are not exported by the host cell to the periplasm or the extracellular space; the polypeptide fragments can be recovered by traditional methods (cf. the discussion below) from the cytoplasm after disruption of the host cells, and if there is need for refolding of the polypeptide fragments, general refolding schemes can be employed, cf. e.g. the disclosure in WO 94/18227 where such a general applicable refolding method is described.

A suitable assay for the potential utility of a given polypeptide fragment derived from SEQ ID NO: 2 is to assess the ability of the polypeptide fragment to effect IFN-γ release from primed memory T-lymphocytes. Polypeptide fragments which have this capability are according to the invention especially interesting embodiments of the invention: It is contemplated that polypeptide fragments which stimulate T lymphocyte immune response shortly after the onset of the infection are important in the control of the mycobacteria causing the infection before the mycobacteria have succeeded in multiplying up to the number of bacteria that would have resulted in fulminant infection.

Thus, an important embodiment of the invention is a polypeptide fragment defined above which

1) induces a release of IFN-γ from primed memory T-lymphocytes withdrawn from a mouse within 2 weeks of primary infection or within 4 days after the mouse has been re-challenge infected with mycobacteria belonging to the tuberculosis complex, the induction performed by the addition of the polypeptide to a suspension comprising about 200,000 spleen cells per ml, the addition of the polypeptide resulting in a concentration of 1-4 μg polypeptide per ml suspension, the release of IFN-γ being assessable by determination of IFN-γ in supernatant harvested 2 days after the addition of the polypeptide to the suspension, and/or

2) induces a release of IFN-γ of at least 300 pg/ml above background level from about 1,000,000 human PBMC (peripheral blood mononuclear cells) per ml isolated from TB patients in the first phase of infection, or from healthy BCG vaccinated donors, or from healthy contacts to TB patients, the induction being performed by the addition of the polypeptide to a suspension comprising the about 1,000,000 PBMC per ml, the addition of the polypeptide resulting in a concentration of 1-4 μg polypeptide per ml suspension, the release of IFN-γ being assessable by determination of IFN-γ in supernatant harvested 2 days after the addition of the polypeptide to the suspension; and/or

3) induces an IFN-γ release from bovine PBMC derived from animals previously sensitized with mycobacteria belonging to the tuberculosis complex, said release being at least two times the release observed from bovine PBMC derived from animals not previously sensitized with mycobacteria belonging to the tuberculosis complex.

The IFN-γ release from bovine PBMC can e.g. be measured as the optical density (OD) index over background in a standard cytokine ELISA and should thus be at least two, but higher numbers such as at least 3, 5, 8, and 10 are preferred.

The polypeptide fragments of the invention preferably comprises an amino acid sequence of at least 12 amino acid residues in length which has a higher sequence identity of at least 80 percent with SEQ ID NO: 2. A preferred minimum percentage of sequence identity is at least 85%, at least 90%, at least 91%, at least 92%, at least 93%, at least 94%, at least 950, at least 96%, at least 97%, at least 98%, at least 99%, and at least 99.5%.

As mentioned above, it will normally be interesting to omit the leader sequences from the polypeptide fragments of the invention. However, by producing fusion polypeptides, superior characteristics of the polypeptide fragments of the invention can be achieved. For instance, fusion partners which facilitate export of the polypeptide when produced recombinantly, fusion partners which facilitate purification of the polypeptide, and fusion partners which enhance the immunogenicity of the polypeptide fragment of the invention are all interesting possibilities. Therefore, the invention also pertains to a fusion polypeptide comprising at least one polypeptide fragment defined above and at least one fusion partner. The fusion partner can, in order to enhance immunogenicity, e.g. be selected from the group consisting of another polypeptide fragment as defined above (so as to allow for multiple expression of relevant epitopes), and an other polypeptide derived from a bacterium belonging to the tuberculosis complex, such as ESAT-6, MPB64, MPT64, and MPB59 or at least one T-cell epitope of any of these antigens. Other immunogenicity enhancing polypeptides which could serve as fusion partners are T-cell epitopes (e.g. derived from the polypeptides ESAT-6, MPB64, MPT64, or MPB59) or other immunogenic epitopes enhancing the immunogenicity of the target gene product, e.g. lymphokines such as INF-γ, IL-2 and IL-12. In order to facilitate expression and/or purification the fusion partner can e.g. be a bacterial fimbrial protein, e.g. the pilus components pilin and papA; protein A; the ZZ-peptide (ZZ-fusions are marketed by Pharmacia in Sweden); the maltose binding protein; gluthatione S-transferase; β-galactosidase; or poly-histidine.

Other interesting fusion partners are polypeptides which are lipidated and thereby effect that the immunogenic polypeptide is presented in a suitable manner to the immune system. This effect is e.g. known from vaccines based on the *Borrelia burgdorferi* OspA polypeptide, wherein the lipidated membrane anchor in the polypeptide confers a self-adjuvating effect to the polypeptide (which is natively lipidated) when isolated from cells producing it. In contrast, the OspA polypeptide is relatively silent immunologically when prepared without the lipidation anchor.

Another part of the invention pertains to a nucleic acid fragment in isolated form which comprises a nucleic acid sequence which encodes a polypeptide or fusion polypeptide as defined above, or comprises a nucleic acid sequence complementary thereto.

It is preferred that the nucleic acid fragment is a DNA fragment.

Such a fragment may be readily prepared by, for example, directly synthesizing the fragment by chemical means, by application of nucleic acid reproduction technology, such as the PCR technology of U.S. Patent 4,603,102, or by introducing selected sequences into recombinant vectors for recombinant production.

It is well known that the same amino acid may be encoded by various codons, the codon usage being related, *inter alia*, to the preference of the organisms in question expressing the nucleotide sequence. Thus, at least one nucleotide or codon of a nucleic acid fragment of the invention may be exchanged by others which, when expressed, result in a polypeptide identical or substantially identical to the polypeptide encoded by the nucleic acid fragment in question. The invention thus allows for variations in the sequence such as substitution, insertion (including introns), addition, deletion and rearrangement of one or more nucleotides, which variations do not have any substantial effect on the polypeptide encoded by the nucleic acid fragment or a subsequence thereof. The term "substitution" is intended to mean the replacement of one or more nucleotides in the full nucleotide sequence with one or more different nucleotides, "addition" is understood to mean the addition of one or more nucleotides at either end of the full nucleotide sequence, "insertion" is intended to mean the introduction of one or more nucleotides within the full nucleotide sequence, "deletion" is intended to indicate that one or more nucleotides have been deleted from the full nucleotide sequence whether at either end of the sequence or at any suitable point within it, and "rearrangement" is intended to mean that two or more nucleotide residues have been exchanged with each other.

The nucleotide sequence to be modified may be of cDNA or genomic origin as discussed above, but may also be of synthetic origin. Furthermore, the sequence may be of mixed cDNA and genomic, mixed cDNA and synthetic or genomic and synthetic origin as discussed above. The sequence may have been modified, e.g. by site-directed mutagenesis, to result in the desired nucleic acid fragment encoding the desired polypeptide. The following discussion focused on modifications of nucleic acid encoding the polypeptide should be understood to encompass also such possibilities, as well as the possibility of building up the nucleic acid by ligation of two or more DNA fragments to obtain the desired nucleic acid fragment, and combinations of the above-mentioned principles.

The nucleotide sequence may be modified using any suitable technique which results in the production of a nucleic acid fragment encoding a polypeptide of the invention.

The modification of the nucleotide sequence encoding the amino acid sequence of the polypeptide of the invention should be one which does not impair the immunological function of the resulting polypeptide.

A preferred method of preparing variants of the antigens disclosed herein is site-directed mutagenesis. This technique is useful in the preparation of individual peptides, or biologically functional equivalent proteins or peptides, derived from the antigen sequences, through specific mutagenesis of the underlying nucleic acid. The technique further provides a ready ability to prepare and test sequence variants, for example, incorporating one or more of the foregoing considerations, by introducing one or more nucleotide sequence changes into the nucleic acid. Site-specific mutagenesis allows the production of mutants through the use of specific oligonucleotide sequences which encode the nucleotide sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to 25 nucleotides in length is preferred, with about 5 to 10 residues on both sides of the junction of the sequence being altered.

In general, the technique of site-specific mutagenesis is well known in the art as exemplified by publications (Adelman et al., 1983). As will be appreciated, the technique typically employs a phage vector which exists in both a single stranded and double stranded form. Typical vectors useful in site-directed mutagenesis include vectors such as the M13 phage (Messing et al., 1981). These phage are readily commercially available and their use is generally well known to those skilled in the art.

In general, site-directed mutagenesis in accordance herewith is performed by first obtaining a single-stranded vector which includes within its sequence a nucleic acid sequence which encodes the polypeptides of the invention. An oligonucleotide primer bearing the desired mutated sequence is prepared, generally synthetically, for example by the method of Crea et al. (1978). This primer is then annealed with the single-stranded vector, and subjected to DNA polymerizing enzymes such as E. coli polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as E. coli cells, and clones are selected which include recombinant vectors bearing the mutated sequence arrangement.

The preparation of sequence variants of the selected nucleic acid fragments of the invention using site-directed mutagenesis is provided as a means of producing potentially useful species of the genes and is not meant to be limiting as there are other ways in which sequence variants of the nucleic acid fragments of the invention may be obtained. For example, recombinant vectors encoding the desired genes may be treated with mutagenic agents to obtain sequence variants (see, e.g., a method described by Eichenlaub, 1979) for the mutagenesis of plasmid DNA using hydroxylamine.

The invention also relates to a replicable expression vector which comprises a nucleic acid fragment defined above, especially a vector which comprises a nucleic acid fragment encoding a polypeptide fragment of the invention.

The vector may be any vector which may conveniently be subjected to recombinant DNA procedures, and the choice of vector will often depend on the host cell into which it is to be introduced. Thus, the vector may be an autonomously replicating vector, *i.e.* a vector which exists as an extrachromosomal entity, the replication of which is independent of chromosomal replication; examples of such a vector are a plasmid, phage, cosmid, mini-chromosome or virus. Alternatively, the vector may be one which, when introduced in a host cell, is integrated in the host cell genome and replicated together with the chromosome(s) into which it has been integrated.

Expression vectors may be constructed to include any of the DNA segments disclosed herein. Such DNA might encode an antigenic protein specific for virulent strains of mycobacteria or even hybridization probes for detecting mycobacteria nucleic acids in samples. Longer or shorter DNA segments could be used, depending on the antigenic protein desired. Epitopic regions of the proteins expressed or encoded by the disclosed DNA could be included as relatively short segments of DNA. A wide variety of expression vectors is possible including, for example, DNA segments encoding reporter gene products useful for identification of heterologous gene products and/or resistance genes such as antibiotic resistance genes which may be useful in identifying transformed cells.

The vector of the invention may be used to transform cells so as to allow propagation of the nucleic acid fragments of the invention or so as to allow expression of the polypeptide fragments of the invention. Hence, the invention also pertains to a transformed cell harbouring at least one such vector according to the invention, said cell being one which does not natively harbour the vector and/or the nucleic acid fragment of the invention contained therein. Such a transformed cell (which is also a part of the invention) may be any suitable bacterial host cell or any other type of cell such as a unicellular eukaryotic organism, a fungus or yeast, or a cell derived from a multicellular organism, e.g. an animal or a plant. It is especially in cases where glycosylation is desired that a mammalian cell is used, although glycosylation of proteins is a rare event in prokaryotes. Normally, however, a prokaryotic cell is preferred such as a bacterium belonging to the genera *Mycobacterium, Salmonella, Pseudomonas, Bacillus* and *Eschericia.* It is preferred that the transformed cell is an *E. coli, B. subtilis,* or *M. bovis* BCG cell, and it is especially preferred that the transformed cell expresses a polypeptide according of the invention. The latter opens for the possibility to produce the polypeptide of the invention by simply recovering it from the culture containing the transformed cell. In the most preferred embodiment of this part of the invention the transformed cell is *Mycobacterium bovis* BCG strain: Danish 1331, which is the *Mycobacterium bovis* strain Copenhagen from the Copenhagen BCG Laboratory, Statens Seruminstitut, Denmark.

The nucleic acid fragments of the invention allow for the recombinant production of the polypeptides fragments of the invention. However, also isolation from the natural source is a way of providing the polypeptide fragments as is peptide synthesis.

Therefore, the invention also pertains to a method for the preparation of a polypeptide fragment of the invention, said method comprising inserting a nucleic acid fragment as defined above into a vector which is able to replicate in a host cell, introducing the resulting recombinant vector into the host cell (transformed cells may be selected using various techniques, including screening by differential hybridization, identification of fused reporter gene products, resistance markers, anti-antigen antibodies and the like), culturing the host cell in a culture medium under conditions sufficient to effect expression of the polypeptide (of course the cell may be cultivated under conditions appropriate to the circumstances, and if DNA is desired, replication conditions are used), and recovering the polypeptide from the host cell or culture medium; or

isolating the polypeptide from a short-term culture filtrate as defined in claim 1; or

isolating the polypeptide from whole mycobacteria of the tuberculosis complex or from lysates or fractions thereof, e.g. cell wall containing fractions, or

synthesizing the polypeptide by solid or liquid phase peptide synthesis.

The medium used to grow the transformed cells may be any conventional medium suitable for the purpose. A suitable vector may be any of the vectors described above, and an appropriate host cell may be any of the cell types listed above. The methods employed to construct the vector and effect introduction thereof into the host cell may be any methods known for such purposes within the field of recombinant DNA. In the following a more detailed description of the possibilities will be given:

In general, of course, prokaryotes are preferred for the initial cloning of nucleic sequences of the invention and constructing the vectors useful in the invention. For example, in addition to the particular strains mentioned in the more specific disclosure below, one may mention by way of example, strains such as *E. coli* K12 strain 294 (ATCC No. 31446), *E. coli* B, and *E. coli* X 1776 (ATCC No. 31537). These examples are, of course, intended to be illustrative rather than limiting.

Prokaryotes are also preferred for expression. The aforementioned strains, as well as *E. coli* W3110 (F-, lambda-, prototrophic, ATCC No. 273325), bacilli such as Bacillus subtilis, or other enterobacteriaceae such as Salmonella typhimurium or Serratia marcesans, and various Pseudomonas species may be used. Especially interesting are rapid-growing mycobacteria, e.g. *M. smegmatis,* as these bacteria have a high degree of resemblance with mycobacteria of the tuberculosis complex and therefore stand a good chance of reducing the need of performing post-translational modifications of the expression product.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species (see, e.g., Bolivar et al., 1977, Gene 2: 95). The pBR322 plasmid contains genes for ampicillin and tetracycline resistance and thus provides easy means for identifying transformed cells. The pBR plasmid, or other microbial plasmid or phage must also contain, or be modified to contain, promoters which can be used by the microorganism for expression.

Those promoters most commonly used in recombinant DNA construction include the B-lactamase (penicillinase) and lactose promoter systems (Chang et al., 1978; Itakura et al., 1977; Goeddel et al., 1979) and a tryptophan (trp) promoter system (Goeddel et al., 1979; EPO Appl. Publ. No. 0036776). While these are the most commonly used, other microbial promoters have been discovered and utilized, and details concerning their nucleotide sequences have been published, enabling a skilled worker to ligate them functionally with plasmid vectors (Siebwenlist et al., 1980). Certain genes from prokaryotes may be expressed efficiently in *E. coli* from their own promoter sequences, precluding the need for addition of another promoter by artificial means.

After the recombinant preparation of the polypeptide according to the invention, the isolation of the polypeptide may for instance be carried out by affinity chromatography (or other conventional biochemical procedures based on chromatography), using a monoclonal antibody which substantially specifically binds the polypeptide according to the invention. Another possibility is to employ the simultaneous electroelution technique described by Andersen et al. in J. Immunol. Methods 161: 29-39.

According to the invention the post-translational modifications involves lipidation, glycosylation, cleavage, or elongation of the polypeptide.

In certain aspects, the DNA sequence information provided by this invention allows for the preparation of relatively short DNA (or RNA or PNA) sequences having the ability to specifically hybridize to mycobacterial gene sequences. In these aspects, nucleic acid probes of an appropriate length are prepared based on a consideration of the relevant sequence. The ability of such nucleic acid probes to specifically hybridize to the mycobacterial gene sequences lend them particular utility in a variety of embodiments. Most importantly, the probes can be used in a variety of diagnostic assays for detecting the presence of pathogenic organisms in a given sample. However, either uses are envisioned, including the use of the sequence information for the preparation of mutant species primers, or primers for use in preparing other genetic constructs.

Apart from their use as starting points for the synthesis of polypeptides of the invention and for hybridization probes (useful for direct hybridization assays or as primers in e.g. PCR or other molecular amplification methods) the nucleic acid fragments of the invention may be used for effecting *in vivo* expression of antigens, *i.e.* the nucleic acid fragments may be used in so-called DNA vaccines. Recent research have revealed that a DNA fragment cloned in a vector which is non-replicative in eukaryotic cells may be introduced into an animal (including a human being) by e.g. intramuscular injection or percutaneous administration (the so-called "gene gun" approach). The DNA is taken up by e.g. muscle cells and the gene of interest is expressed by a promoter which is functioning in eukaryotes, e.g. a viral promoter, and the gene product thereafter stimulates the immune system. These newly discovered methods are reviewed in Ulmer et al., 1993, which hereby is included by reference.

Hence, the invention also relates to a vaccine comprising a nucleic acid fragment according to the invention, the vaccine effecting *in vivo* expression of antigen by an animal, including a human being, to whom the vaccine has been administered, the amount of expressed antigen being effective to confer substantially increased resistance to infections with mycobacteria of the tuberculosis complex in an animal, including a human being.

The efficacy of such a "DNA vaccine" can possibly be enhanced by administering the gene encoding the expression product together with a DNA fragment encoding a polypeptide which has the capability of modulating an immune response. For instance, a gene encoding lymphokine precursors or lymphokines (e.g. IFN-γ, IL-2, or IL-12) could be administered together with the gene encoding the immunogenic protein, either by administering two separate DNA fragments or by administering both DNA fragments included in the same vector. It also is a possibility to administer DNA fragments comprising a multitude of nucleotide sequences which each encode relevant epitopes of the polypeptides disclosed herein so as to effect a continuous sensitization of the immune system with a broad spectrum of these epitopes.

As explained above, the polypeptide fragments of the invention are excellent candidates for vaccine constituents or for constituents in an immune diagnostic agent due to their extracellular presence in culture media containing metabolizing virulent mycobacteria belonging to the tuberculosis complex, or because of their high homologies with such extracellular antigens, or because of their absence in *M. bovis* BCG.

Thus, another part of the invention pertains to an immunologic composition comprising a polypeptide or fusion polypeptide according to the invention. In order to ensure optimum performance of such an immunologic composition it is preferred that it comprises an immunologically and pharmaceutically acceptable carrier, vehicle or adjuvant.

Suitable carriers are selected from the group consisting of a polymer to which the polypeptide(s) is/are bound by hydrophobic non-covalent interaction, such as a plastic, e.g. polystyrene, or a polymer to which the polypeptide(s) is/are covalently bound, such as a polysaccharide, or a polypeptide, e.g. bovine serum albumin, ovalbumin or keyhole limpet haemocyanin. Suitable vehicles are selected from the group consisting of a diluent and a suspending agent. The adjuvant is preferably selected from the group consisting of dimethyldioctadecylammonium bromide (DDA), Quil A, poly I:C, Freund's incomplete adjuvant, IFN-γ, IL-2, IL-12, monophosphoryl lipid A (MPL), and muramyl dipeptide (MDP).

A preferred immunologic composition according to the present invention comprising at least two different polypeptide fragments, each different polypeptide fragment being a polypeptide or a fusion polypeptide defined above. It is preferred that the immunologic composition comprises between 3-20 different polypeptide fragments or fusion polypeptides.

Such an immunologic composition may preferably be in the form of a vaccine or in the form of a skin test reagent.

In line with the above, the invention therefore also pertain to a method for producing an immunologic composition according to the invention, the method comprising preparing, synthesizing or isolating a polypeptide according to the invention, and solubilizing or dispersing the polypeptide in a medium for a vaccine, and optionally adding other *M. tuberculosis* antigens and/or a carrier, vehicle and/or adjuvant substance.

Preparation of vaccines which contain peptide sequences as active ingredients is generally well understood in the art, as exemplified by U.S. Patents 4,608,251; 4,601,903; 4,599,231; 4,599,230; 4,596,792; and 4,578,770, all incorporated herein by reference. Typically, such vaccines are prepared as injectables either as liquid solutions or suspensions; solid forms suitable for solution in, or suspension in, liquid prior to injection may also be prepared. The preparation may also be emulsified. The active immunogenic ingredient is often mixed with excipients which are pharmaceutically acceptable and compatible with the active ingredient. Suitable excipients are, for example, water, saline, dextrose, glycerol, ethanol, or the like, and combinations thereof. In addition, if desired, the vaccine may contain minor amounts of auxiliary substances such as wetting or emulsifying agents, pH buffering agents, or adjuvants which enhance the effectiveness of the vaccines.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkalene glycols or triglycerides; such suppositories may be formed from mixtures containing the active ingredient in the range of 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95% of active ingredient, preferably 25-70%.

The proteins may be formulated into the vaccine as neutral or salt forms. Pharmaceutically acceptable salts include acid addition salts (formed with the free amino groups of the peptide) and which are formed with inorganic acids such as, for example, hydrochloric or phosphoric acids, or such organic acids as acetic oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, including, e.g., the capacity of the individual's immune system to mount an immune response, and the degree of protection desired. Suitable dosage ranges are of the order of several hundred micrograms active ingredient per vaccination with a preferred range from about 0.1 $\mu$g to 1000 $\mu$g, such as in the range from about 1 $\mu$g to 300 $\mu$g, and especially in the range from about 10 $\mu$g to 50 $\mu$g. Suitable regimens for initial administration and booster shots are also variable but are typified by an initial administration followed by subsequent inoculations or other administrations.

The manner of application may be varied widely. Any of the conventional methods for administration of a vaccine are applicable. These are believed to include oral application on a solid physiologically acceptable base or in a physiologically acceptable dispersion, parenterally, by injection or the like. The dosage of the vaccine will depend on the route of administration and will vary according to the age of the person to be vaccinated and, to a lesser degree, the size of the person to be vaccinated.

Some of the polypeptides of the vaccine are sufficiently immunogenic in a vaccine, but for some of the others the immune response will be enhanced if the vaccine further comprises an adjuvant substance.

Various methods of achieving adjuvant effect for the vaccine include use of agents such as aluminum hydroxide or phosphate (alum), commonly used as 0.05 to 0.1 percent solution in phosphate buffered saline, admixture with synthetic polymers of sugars (Carbopol) used as 0.25 percent solution, aggregation of the protein in the vaccine by heat treatment with temperatures ranging between 70° to 101°C for 30 second to 2 minute periods respectively. Aggregation by reactivating with pepsin treated (Fab) antibodies to albumin, mixture with bacterial cells such as C. parvum or endotoxins or lipopolysaccharide components of gram-negative bacteria, emulsion in physiologically acceptable oil vehicles such as mannide monooleate (Aracel A) or emulsion with 20 percent solution of a perfluorocarbon (Fluosol-DA) used as a block substitute may also be employed. According to the invention DDA (dimethyldioctadecylammonium bromide) is an interesting candidate for an adjuvant, but also Freund's complete and incomplete adjuvants as well as QuilA and RIBI are interesting possibilities. Further possibilities are monophosphoryl lipid A (MPL), and muramyl dipeptide (MDP).

Another highly interesting (and thus, preferred) possibility of achieving adjuvant effect is to employ the technique described in Gosselin *et al.,* 1992 (which is hereby incorporated by reference herein). In brief, the presentation of a relevant antigen such as an antigen of the present invention can be enhanced by conjugating the antigen to antibodies (or antigen binding antibody fragments) against the Fcy receptors on monocytes/macrophages. Especially conjugates between antigen and anti-Fc$\gamma$RI have been demonstrated to enhance immunogenicity for the purposes of vaccination.

Other possibilities involve the use of immune modulating substances such as lymphokines (e.g. IFN-$\gamma$, IL-2

and IL-12) or synthetic IFN-γ inducers such as poly I:C in combination with the above-mentioned adjuvants. As discussed in example 3, it is contemplated that such mixtures of antigen and adjuvant will lead to superior vaccine formulations.

In many instances, it will be necessary to have multiple administrations of the vaccine, usually not exceeding six vaccinations, more usually not exceeding four vaccinations and preferably one or more, usually at least about three vaccinations. The vaccinations will normally be at from two to twelve week intervals, more usually from three to five week intervals. Periodic boosters at intervals of 1-5 years, usually three years, will be desirable to maintain the desired levels of protective immunity. The course of the immunization may be followed by *in vitro* proliferation assays of PBL (peripheral blood lymphocytes) co-cultured with ESAT-6 or ST-CF, and especially by measuring the levels of IFN-γ released form the primed lymphocytes. The assays may be performed using conventional labels, such as radionuclides, enzymes, fluorescers, and the like. These techniques are well known and may be found in a wide variety of patents, such as U.S. Patent Nos. 3,791,932; 4,174,384 and 3,949,064, as illustrative of these types of assays.

Due to genetic variation, different individuals may react with immune responses of varying strength to the same polypeptide. Therefore, the vaccine according to the invention may comprise several different polypeptides in order to increase the immune response. The vaccine may comprise two or more polypeptides, where all of the polypeptides are as defined above, or some but not all of the peptides may be derived from a bacterium belonging to the *M. tuberculosis* complex. In the latter example the polypeptides not necessarily fulfilling the criteria set forth above for polypeptides may either act due to their own immunogenicity or merely act as adjuvants. Examples of such interesting polypeptides are MPB64, MPT64, and MPB59, but any other substance which can be isolated from mycobacteria are possible candidates.

The vaccine may comprise 3-20 different polypeptides, such as 3-10 different polypeptides.

One reason for admixing the polypeptides of the invention with an adjuvant is to effectively activate a cellular immune response. However, this effect can also be achieved in other ways, for instance by expressing the effective antigen in a vaccine in a non-pathogenic microorganism. A well-known example of such a microorganism is *Mycobacterium bovis* BCG.

Therefore, another important aspect of the present invention is an improvement of the living BCG vaccine presently available, which is a vaccine for immunizing an animal, including a human being, against TB caused by mycobacteria belonging to the tuberculosis-complex, comprising as the effective component a microorganism, wherein one or more copies of a DNA sequence encoding a polypeptide as defined above has been incorporated into the genome of the microorganism in a manner allowing the microorganism to express and secrete the polypeptide.

In the present context the term "genome" refers to the chromosome of the microorganisms as well as extrachromosomally DNA or RNA, such as plasmids. It is, however, preferred that the DNA sequence of the present invention has been introduced into the chromosome of the non-pathogenic microorganism, since this will prevent loss of the genetic material introduced.

It is preferred that the non-pathogenic microorganism is a bacterium, e.g. selected from the group consisting of the genera *Mycobacterium*, *Salmonella, Pseudomonas* and *Eschericia*. It is especially preferred that the non-pathogenic microorganism is *Mycobacterium bovis* BCG, such as *Mycobacterium bovis* BCG strain: Danish 1331.

The incorporation of one or more copies of a nucleotide sequence encoding the polypeptide according to the invention in a mycobacterium from a *M. bovis* BCG strain will enhance the immunogenic effect of the BCG strain. The incorporation of more than one copy of a nucleotide sequence of the invention is contemplated to enhance the immune response even more, and consequently an aspect of the invention is a vaccine wherein at least 2 copies of a DNA sequence encoding, a polypeptide is incorporated in the genome of the microorganism, such as at least 5 copies. The copies of DNA sequences may either be identical encoding identical polypeptides or be variants of the same DNA sequence encoding identical or homologues of a polypeptide, or in another embodiment be different DNA sequences encoding different polypeptides where at least one of the polypeptides is according to the present invention.

The living vaccine of the invention can be prepared by cultivating a transformed non-pathogenic cell according to the invention, and transferring these cells to a medium for a vaccine, and optionally adding a carrier, vehicle and/or adjuvant substance.

When diagnosis of previous or ongoing infection with virulent mycobacteria is the aim, a blood sample comprising mononuclear cells (i.a. T-lymphocytes) from a patient could be contacted with a sample of one or more polypeptides of the invention. This contacting can be performed *in vitro* and a positive reaction could e.g. be proliferation of the T-cells or release cytokines such as γ-interferon into the extracellular phase (e.g. into a culture supernatant); a suitable *in vivo* test would be a skin test as described above. It is also, conceivable to contact a serum sample from a subject to contact with a polypeptide of the invention, the demonstration of a binding between antibodies in the serum sample and the polypeptide being indicative of previous or ongoing infection.

The invention therefore also relates to an *in vitro* method for diagnosing ongoing or previous sensitization in an animal or a human being with bacteria belonging to the tuberculosis complex, the method comprising providing a blood sample from the animal or human being, and contacting the sample from the animal with the polypeptide of the invention, a significant release into the extracellular phase of at least one cytokine by mononuclear cells in the blood sample being indicative of the animal being sensitized. By the term "significant release" is herein meant that the release of the cytokine is significantly higher than the cytokine release from a blood sample derived from a non-tuberculous subject (e.g. a subject which does not react in a traditional skin test for TB). Normally, a significant release is at least two times the release observed from such a sample.

Finally, a monoclonal or polyclonal antibody, which is specifically reacting with a polypeptide of the invention in an immuno assay, or a specific binding fragment of said antibody, is also a part of the invention. The production of such polyclonal antibodies requires that a suitable animal be immunized with the polypeptide and that these antibodies are subsequently isolated, suitably by immune affinity chromatography. The production of monoclonals can be effected by methods well-known in the art, since the present invention provides for adequate amounts of antigen for both immunization and screening of positive hybridomas.

LEGENDS TO THE FIGURES

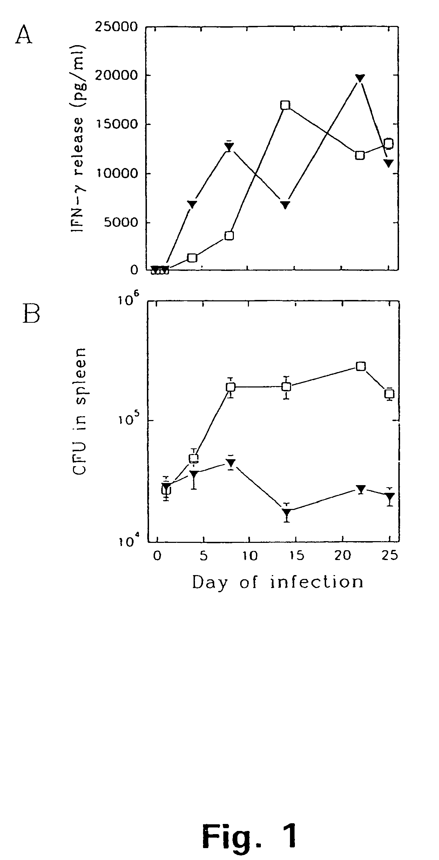

Fig. 1: Long term memory immune mice are very efficiently protected towards an infection with *M. tuberculosis.* Mice were given a challenge of *M. tuberculosis* and spleens were isolated at different time points. Spleen lymphocytes were stimulated *in vitro* with ST-CF and the release of IFN-γ investigated (panel A). The counts of CFU in the spleens of the two groups of mice are indicated in panel B. The memory immune mice control infection within the first week and produce large quantities of IFN-γ in response to antigens in ST-CF.

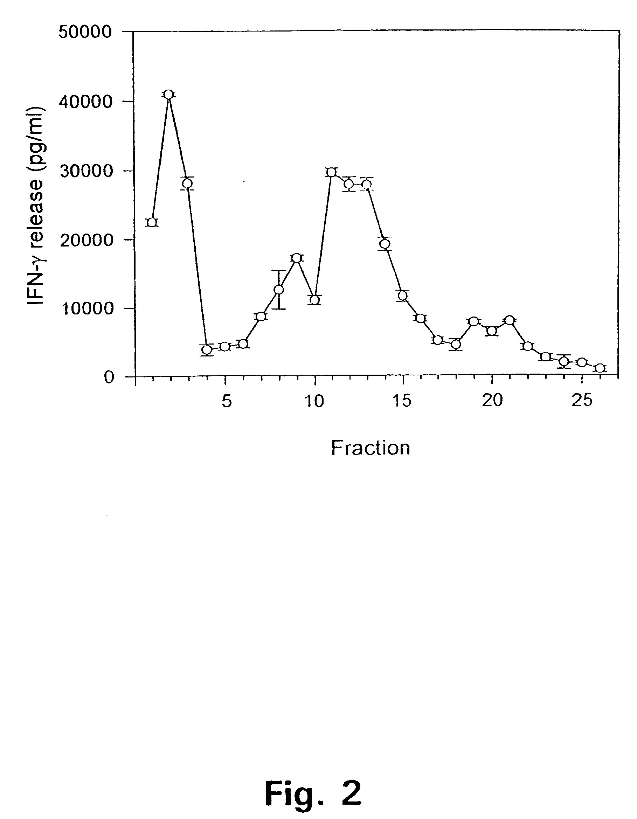

Fig. 2: T cells involved in protective immunity are predominantly directed to molecules from 6-12 and 17-38 kDa. Splenic T cells were isolated four days after the challenge with *M. tuberculosis* and stimulated *in vitro* with narrow molecular mass fractions of ST-CF. The release of IFN-γ was investigated

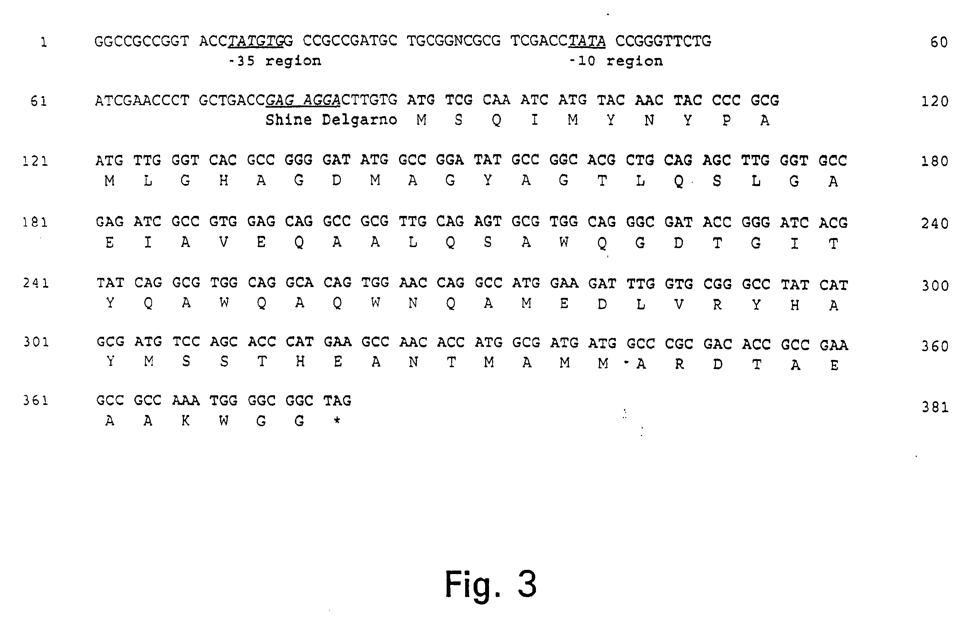

Fig. 3: Nucleotide sequence (SEQ ID NO: 1) of *cfp7*. The deduced amino acid sequence (SEQ ID NO: 2) of CFP7 is given in conventional one-letter code below the nucleotide sequence. The putative ribosome-binding site is written in underlined italics as are the putative -10 and -35 regions. Nucleotides written in bold are those encoding CFP7.

EXAMPLE 1

*Identification of single culture filtrate antigens involved in protective immunity*

A group of efficiently protected mice was generated by infecting 8-12 weeks old female C57Bl/6j mice with 5 x 10⁴ *M. tuberculosis* i.v. After 30 days of infection the mice were subjected to 60 days of antibiotic treatment with isoniazid and were then left for 200-240 days to ensure the establishment of resting long-term memory immunity. Such memory immune mice are very efficiently protected against a secondary infection (Fig. 1). Long lasting immunity in this model is mediated by a population of highly reactive CD4 cells recruited to the site of infection and triggered to produce large amounts of IFN-γ in response to ST-CF (Fig. 1) (Andersen *et al.* 1995).

We have used this model to identify single antigens recognized by protective T cells. Memory immune mice were reinfected with 1 x 10⁶ *M. tuberculosis* i.v. and splenic lymphocytes were harvested at day 4-6 of reinfection, a time point where this population is highly reactive to ST-CF. The antigens recognized by these T cells were mapped by the multi-elution technique (Andersen and Heron, 1993). This technique divides complex protein mixtures separated in SDS-PAGE into narrow fractions in a physiological buffer. These fractions were used to stimulate spleen lymphocytes *in vitro* and the release of IFN-γ was monitored (Fig. 2). Long-term memory immune mice did not recognize these fractions before TB infection, but splenic lymphocytes obtained during the recall of protective immunity recognized a range of culture filtrate antigens and peak production of IFN-γ was found in response to proteins of apparent molecular weight 6-12 and 17-30 kDa (Fig. 2). It is therefore concluded that culture filtrate antigens within these regions are the major targets recognized by memory effector T-cells triggered to release IFN-γ during the first phase of a protective immune response.

EXAMPLE 2

*Cloning of genes expressing low mass culture filtrate antigens*

In example 1 it was demonstrated that antigens in the low molecular mass fraction are recognized strongly by cells isolated from memory immune mice. Monoclonal antibodies (mAbs) to these antigens were therefore generated by immunizing with the low mass fraction in RIBI adjuvant (first and second immunization) followed by two injections with the fractions in aluminium hydroxide. Fusion and cloning of the reactive cell lines were done according to standard procedures (Kohler and Milstein 1975). The procedure resulted in the provision of two mAbs: ST-3 directed to a 9 kDa culture filtrate antigen (CFP9) and PV-2 directed to a 7 kDa antigen (CFP7), when the molecular weight is estimated from migration of the antigens in an SDS-PAGE.

In order to identify the antigens binding to the Mab's, the following experiments were carried out:

The recombinant λgt11 *M. tuberculosis* DNA library constructed by R. Young (Young, R.A. *et al.* 1985) and obtained through the World Health Organization IMMTUB programme (WHO.0032.wibr) was screened for phages expressing gene products which would bind the monoclonal antibodies ST-3 and PV-2.

Approximately 1 x $10^5$ pfu of the gene library (containing approximately 25% recombinant phages) were plated on *Eschericia coli* Y1090 (DlacU169, proA+, Dlon, araD139, supF, trpC22::tn10 [pMC9] ATCC#37197) in soft agar and incubated for 2,5 hours at 42°C.

The plates were overlaid with sheets of nitrocellulose saturated with isopropyl-β-D-thiogalactopyranoside and incubation was continued for 2,5 hours at 37°C. The nitrocellulose was removed and incubated with samples of the monoclonal antibodies in PBS with Tween 20 added to a final concentration of 0.05%. Bound monoclonal antibodies were visualized by horseradish peroxidase-conjugated rabbit anti-mouse immunoglobulins (P260, Dako, Glostrup, DK) and a staining reaction involving 5,5',3,3'-tetramethylbenzidine and $H_2O_2$.

Positive plaques were recloned and the phages originating from a single plaque were used to lysogenize *E. coli* Y1089 (DlacU169, proA+, Dlon, araD139, strA, hf1150 [chr::*tn10*] [pMC9] ATCC nr. 37196). The resultant lysogenic strains were used to propagate phage particles for DNA extraction. These lysogenic *E. coli* strains have been named:

AA226 (expressing ST-3 reactive polypeptide CFP9) which has been deposited 28 June 1993 with the collection of Deutsche Sammlung von Mikroorganismen und Zellkulturen GmbH (DSM) under the accession number DSM 8377 and in accordance with the provisions of the Budapest Treaty, and

AA242 (expressing PV-2 reactive polypeptide CFP7) which has been deposited 28 June 1993 with the collection of Deutsche Sammlung von Mikroorganismen und Zellkulturen GmbH (DSM) under the accession number DSM 8379 and in accordance with the provisions of the Budapest Treaty.

These two lysogenic *E. coli* strains are disclosed in WO 95/01441 as are the mycobacterial polypeptide products expressed thereby. However, no information concerning the amino acid sequences of these polypeptides or their genetic origin are given, and therefore only the direct expression products of AA226 and AA242 are made available to the public.

The st-3 binding protein is expressed as a protein fused to β-galactosidase, whereas the pv-2 binding protein appears to be expressed in an unfused version.

Sequencing of the nucleotide sequence encoding the PV-2 and ST-3 binding protein

In order to obtain the nucleotide sequence of the gene encoding the pv-2 binding protein, the approximately 3 kb *M. tuberculosis* derived *Eco*RI - *Eco*RI fragment from AA242 was subcloned in the EcoRI site in the pBluescriptSK + (Stratagene) and used to transform *E. coli* XL-1Blue (Stratagene).

Similarly, to obtain the nucleotide sequence of the gene encoding the st-3 binding protein, the approximately 5 kb *M. tuberculosis* derived *Eco*RI - *Eco*RI fragment from AA226 was subcloned in the EcoRI site in the pBluescriptSK + (Stratagene) and used to transform *E. coli* XL-1Blue (Stratagene).

The complete DNA sequence of both genes were obtained by the dideoxy chain termination method adapted for supercoiled DNA by use of the Sequenase DNA sequencing kit version 1.0 (United States Biochemical Corp., Cleveland, OH) and by cycle sequencing using the Dye Terminator system in combination with an automated gel reader (model 373A; Applied Biosystems) according to the instructions provided. The sequences DNA are shown in SEQ ID NO: 1 (CFP7) and in SEQ ID NO: 3 (CFP9) as well as in Figs. 3 and 4, respectively. Both strands of the DNA were sequenced.

CFP7

An open reading frame (ORF) encoding a sequence of 96 amino acid residues was identified from an ATG start codon at position 91-93 extending to a TAG stop codon at position 379-381. The deduced amino acid sequence is shown in SEQ ID NO: 2 (and in Fig. 3 where conventional one-letter amino acid codes are used).

CFP7 appear to be expressed in *E. coli* as an unfused version. The nucleotide sequence at position 78-84 is expected to be the Shine Delgarno sequence and the sequences from position 47-50 and 14-19 are expected to be the -10 and -35 regions, respectively:

Subcloning CFP7 in expression vectors

The ORF encoding CFP7 was PCR cloned into the pMST24 (Theisen *et al.*, 1995) expression vector pRVN01.

The PCR amplification was carried out in a thermal reactor (Rapid cycler, Idaho Technology, Idaho) by mixing 10 ng plasmid DNA with the mastermix (0.5 $\mu$M of each oligonucleotide primer, 0.25 $\mu$M BSA (Stratagene), low salt buffer (20 mM Tris-HCl, pH 8.8, 10 mM KCl, 10 mM $(NH_4)_2SO_4$, 2 mM $MgSO_4$ and 0,1% Triton X-100) (Stratagene), 0.25 mM of each deoxynucleoside triphosphate and 0.5 U Taq Plus Long DNA polymerase (Stratagene)). Final volume was 10 $\mu$l (all concentrations given are concentrations in the final volume). Predenaturation was carried out at 94°C for 30 s. 30 cycles of the following was performed; Denaturation at 94°C for 30 s, annealing at 55°C for 30 s and elongation at 72°C for 1 min.

The oligonucleotide primers were synthesised automatically on a DNA synthesizer (Applied Biosystems, Forster City, Ca, ABI-391, PCR-mode), deblocked, and purified by ethanol precipitation.

The *cfp7* oligonucleotides (TABLE 1) were synthesised on the basis of the nucleotide sequence from the CFP7 sequence (Fig. 3). The oligonucleotides were engineered to include an *Sma*I restriction enzyme site at the 5' end and a BamHI restriction enzyme site at the 3' end for directed subcloning.

CFP7

By the use of PCR a *Sma*I site was engineered immediately 5' of the first codon of the ORF of 291 bp, encoding the *cfp7* gene, so that only the coding region would be expressed, and a *Bam*BI site was incorporated right after the stop codon at the 3' end. The 291 bp PCR fragment was cleaved by *Sma*I and *Bam*HI, purified from an agarose gel and subcloned into the *Sma*I - *Bam*HI sites of the pMST24 expression vector. Vector DNA containing the gene fusion was used to transform the *E. coli* XL1-Blue (pRVN01).

Purification of recombinant CFP7

The ORF was fused N-terminally to the $(His)_6$-tag (cf. EP-A-0 282 242). Recombinant antigen was prepared as follows: Briefly, a single colony of *E. coli* harbouring either the pRVN01 or the pRVN02 plasmid, was inoculated into Luria-Bertani broth containing 100 $\mu$g/ml ampicillin and 12.5 $\mu$g/ml tetracycline and grown at 37°C to $OD_{600nm}$ = 0.5. IPTG (isopropyl-$\beta$-D-thiogalactoside) was then added to a final concentration of 2 mM (expression was regulated either by the strong IPTG inducible $P_{tac}$ or the T5 promoter) and growth was continued for further 2 hours. The cells were harvested by centrifugation at 4,200 x g at 4°C for 8 min. The pelleted bacteria were stored overnight at -20°C. The pellet was resuspended in BC 40/100 buffer (20 mM Tris-HCl pH 7.9, 20% glycerol, 100 mM KCl, 40 mM Imidazole) and cells were broken by sonication (5 times for 30 s with intervals of 30 s) at 4°C. followed by centrifugation at 12,000 x g for 30 min at 4°C, the supernatant (crude extract) was used for purification of the recombinant antigens.

The Histidine fusion protein (His-rCFP7) was purified from the crude extract by affinity chromatography on a $Ni^{2+}$-NTA column from QIAGEN with a volume of 100 ml. His-rCFP7 and His-rCFP9 binds to $Ni^{2+}$. After extensive washes of the column in BC 40/100 buffer, the fusion protein was eluted with a BC 1000/100 buffer containing 100 mM imidazole, 20 mM Tris pH 7.9, 20% glycerol and 1 M KCl. subsequently, the purified products were dialysed extensively against 10 mM Tris pH 8.0. His-rCFP7 and His-rCFP9 were then separated from contaminants by fast protein liquid chromatography (FPLC) over an anion-exchange column (Mono Q, Pharmacia, Sweden) in 10 mM Tris pH 8.0 with a linear gradient of NaCl from 0 to 1 M. Aliquots of the fractions were analyzed by 10%-20% gradient sodium dodecyl sulphate polyacrylamide gel electrophoresis (SDS-PAGE). Fractions containing purified His-rCFP7 were pooled.

TABLE 1. Sequence of the *cfp7* oligonucleotides[a].

| Orientation and oligonucleotide | Sequences (5' → 3') | Position[b] (nucleotide) |
|---|---|---|
| Sense | | |
| pvR3 | <u>GCAACACCCGGG</u>ATGTCGCAAATCATG (SEQ ID NO: 43) | 91-105 (SEQ ID NO: 1) |
| Antisense | | |

(continued)

| Orientation and oligonucleotide | Sequences (5' → 3') | Position[b] (nucleotide) |
|---|---|---|
| pvF4 | CTACTAAGCTTGGATCC CTAGCCGCCCCATTTGGCGG (SEQ ID NO: 45) | 381-362 (SEQ ID NO: 1) |

[a] The *cfp7* oligonucleotides were based on the nucleotide sequence shown in Fig. 3 (SEQ ID NO: 1). Nucleotides underlined are not contained in the nucleotide sequence of *cfp7*. [b] The positions referred to are of the non-underlined part of the primers and correspond to the nucleotide sequence shown in Fig. 3.

EXAMPLE 2

*Mapping of the purified antigen in a 2DE system.*

In order to characterize the purified antigen it was mapped in a 2-dimensional electrophoresis (2DE) reference system. This consists of a silver stained gel containing ST-CF proteins separated by isoelectrical focusing followed by a separation according to size in a polyacrylamide gel electrophoresis. The 2DE was performed according to Hochstrasser *et al.* (1988). 85 μg of ST-CF was applied to the isoelectrical focusing tubes where BioRad ampholytes BioLyt 4-6 (2 parts) and BioLyt 5-7 (3 parts) were included. The first dimension was performed in acrylamide/piperazin diacrylamide tube gels in the presence of urea, the detergent CHAPS and the reducing agent DTT at 400 V for 18 hours and 800 V for 2 hours. The second dimension 10-20% SDS-PAGE was performed at 100 V for 18 hours and silver stained. The identification of CFP7 in the 2DE reference gel were done by comparing the spot pattern of the purified antigen with ST-CF with and without the purified antigen. By the assistance of an analytical 2DE software system (Phoretix International, UK) the spots have been identified in Fig. 6.

*Biological activity of the purified recombinant antigen.*

Interferon-γ induction in the mouse model of TB infection.

**Primary infections.** 8 to 12 weeks old female C57BL/6j(H-2[b]), CBA/J(H-2[k]), DBA.2(H-2[d]) and A.SW(H-2[s]) mice (Bomholtegaard, Ry) were given intravenous infections via the lateral tail vein with an inoculum of $5 \times 10^4$ *M. tuberculosis* suspended in PBS in a vol. of 0.1 ml. 14 days postinfection the animals were sacrificed and spleen cells were isolated and tested for the recognition of recombinant antigen.

**Memory responses.** 8-12 weeks old female C57HL/6j(H-2[b]) mice (Bomholtegaard, Ry) were given intravenous infections via the lateral tail vein with an inoculum of $5 \times 10^4$ *M. tuberculosis* suspended in PBS in a vol. of 0.1 ml. After 1 month of infection the mice were treated with isoniazid (Merck and Co., Rahway, NJ) and rifabutin (Farmatalia Carlo Erba, Milano, Italy) in the drinking water, for two months. The mice were rested for 4-6 months before being used in experiments. For the study of the recall of memory immunity, animals were infected with an inoculum of $1 \times 10^6$ bacteria i.v. and sacrificed at day 4 postinfection. Spleen cells were isolated and tested for the recognition of recombinant antigen. Stimulation with rCFP7, resulted in an IFN-γ no higher than 1/3 of the response seen with ST-CF. IFN-γ producing cells. The antigen did not stimulate IFN-γ release in naive mice. Additionally it was non toxic to the cell cultures.

**TABLE 7.** T cell responses in primary TB infection.

| Name | c57BL/6J (H2[b]) | DBA.2 (H2[d]) | CBA/J (H2[k]) | A.SW (H2[s]) |
|---|---|---|---|---|
| rCFP7 | + | + | - | - |

Mouse IFN-γ release during recall of memory immunity to *M. tuberculosis*. -:no response; +: 1/3 of ST-CF; ++: 2/3 of ST-CF; +++: level of ST-CF.

**TABLE 8.** T cell responses in memory immune animals.

| Name | Memory response |
|---|---|
| rCFP7 | + |

Mouse IFN-γ release 14 days after primary infection with *M. tuberculosis*.
-:no response; +: 1/3 of ST-CF; ++: 2/3 of ST-CF; +++: level of ST-CF.

Interferon-γ induction in human TB patients and BCG vaccinated people.

**Human donors:** PBMC were obtained from healthy BCG vaccinated donors with no known exposure to patients with TB and from patients with culture or microscopy proven infection with *Mycobacterium tuberculosis*. Blood samples were drawn from the TB patients 1-4 months after diagnosis.

**Lymphocyte preparations and cell culture:** PBMC were freshly isolated by gradient centrifugation of heparinized blood on Lymphoprep (Nycomed, Oslo, Norway). The cells were resuspended in complete medium: RPMI 1640 (Gibco, Grand Island, N.Y.) supplemented with 40 μg/ml streptomycin, 40 U/ml penicillin, and 0.04 mM/ml glutamine, (all from Gibco Laboratories, Paisley, Scotland) and 10% normal human ABO serum (NHS) from the local blood bank. The number and the viability of the cells were determined by trypan blue staining. Cultures were established with 2,5 x $10^5$ PBMC in 200 μl in microtitre plates (Nunc, Roskilde, Denmark) and stimulated with no antigen, ST-CF, PPD (2.5 μg/ml); rCFP7, in a final concentration of 5 μg/ml. Phytohaemagglutinin, 1 μg/ml (PHA, Difco laboratories, Detroit, MI. was used as a positive control. Supernatants for the detection of cytokines were harvested after 5 days of culture, pooled and stored at -80°C until use.

**Cytokine analysis:** Interferon-γ (IFN-γ ) was measured with a standard ELISA technique using a commercially available pair of mAb's from Endogen and used according to the instructions for use. Recombinant IFN-γ (Gibco laboratories) was used as a standard. The detection level for the assay was 50 pg/ml. The variation between the duplicate wells did not exceed 10 % of the mean. Responses of 9 individual donors are shown in TABLE 9.

A seen in TABLE 9 high levels of IFN-γ release are obtained after stimulation with several of the recombinant antigens. rCFP7 seems to be most strongly recognized by BCG vaccinated healthy donors.

**TABLE 9.** Mean values of results from the stimulation of human blood cells from 7 BCG vaccinated and 7 TB patients with recombinant antigens. SE values are given for each antigen. ST-CF and *M. avium* culture filtrate are shown for the comparison.

Controls, Healthy, BCG vaccinated, no known TB exposure

| donor: | no ag | PHA | PPD | STCF | CFP7 |
|---|---|---|---|---|---|
| 1 | 6 | 9564 | 6774 | 3966 | 7034 |
| 2 | 48 | 12486 | 6603 | 8067 | 3146 |
| 3 | 190 | 11929 | 10000 | 8299 | 8015 |
| 4 | 10 | 21029 | 4106 | 3537 | 1323 |
| 5 | 1 | 18750 | 14209 | 13027 | 17725 |

TB patients, 1-4 month after diagnosis

| | no ag | PHA | PPD | STCF | CFP7 |
|---|---|---|---|---|---|
| 6 | 9 | 8973 | 5096 | 6145 | 852 |
| 7 | 1 | 12413 | 6281 | 3393 | 168 |
| 8 | 4 | 11915 | 7671 | 7375 | 104 |
| 9 | 32 | 22130 | 16417 | 17213 | 8450 |

EXAMPLE 3

The recombinant antigens were tested individually as subunit vaccines in mice. Five groups of 6-8 weeks old, female C57B1/6j mice (Bomholtegard, Denmark) were immunized subcutaneously at the base of the tail with vaccines of the following composition:

Group 1: 10 μg CFP7

Group 2: 50 µg ST-CF
Group 3: Adjuvant control group
Group 4: BCG 2,5 x $10^5$/ml, 0,2 ml
Group 5: Control group: Untreated

The subunit vaccine was given with DDA as adjuvant. The animals were vaccinated with a volume of 0.2 ml. Two weeks after the first injection and three weeks after the second injection group 1-9 were boosted a little further up the back. One week after the last injection the mice were bled and the blood cells were isolated. The immune response induced was monitored by release of IFN-γ into the culture supernatant when stimulated in vitro with the homologous protein.

6 weeks after the last immunization the mice were aerosol challenged with 5 x $10^6$ viable *Mycobacterium tuberculosis*/ml. After 6 weeks of infection the mice were killed and the number of viable bacteria in lung and spleen of infected mice was determined by plating serial 3-fold dilutions of organ homogenates on 7H11 plates. Colonies were counted after 2-3 weeks of incubation. The protective efficacy is expressed as the difference between $log_{10}$ values of the geometric mean of counts obtained from five mice of the relevant group and the geometric mean of counts obtained from five mouse of the relevant control group.

The results from the experiments are presented in the following table.

| Immunogenicity and protective efficacy in mice, of ST-CF and subunit vaccine | | |
|---|---|---|
| Subunit Vaccine | Immunogenicity | Protective efficacy |
| ST-CF | +++ | +++ |
| CFP7 | ++ | - |
| +++ Strong immunogen / high protection (level of BCG) | | |
| ++ Medium immunogen / medium protection | | |
| - No recognition / no protection | | |

In conclusion, we have identified a protein inducing high levels of protection. CFP7 did not induce protection in the mouse model.

EXAMPLE 4

*Species distribution of cfp7.*

Presence of *cfp7*, in different mycobacterial species.

In order to determine the distribution of the *cfp7* gene in species belonging to the M. tuberculosis-complex and in other mycobacteria PCR and/or Southern blotting was used. The bacterial strains used are listed in TABLE 10. Genomic DNA was prepared from mycobacterial cells as described previously (Andersen et al. 1992).

PCR analyses were used in order to determine the distribution of the *cfp7* gene in species belonging to the tuberculosis-complex and in other mycobacteria. The bacterial strains used are listed in TABLE 10. PCR was performed on genomic DNA prepared from mycobacterial cells as described previously (Andersen *et al.*, 1992).

The oligonucleotide primers used were synthesised automatically on a DNA synthesizer (Applied Biosystems, Forster City, Ca, ABI-391, PCR-mode), deblocked, and purified by ethanol precipitation. The primers used for the analyses are shown in

TABLE 11.

The PCR amplification was carried out in a thermal reactor (Rapid cycler, Idaho Technology, Idaho) by mixing 20 ng chromosomal with the mastermix (contained 0.5 µM of each oligonucleotide primer, 0.25 µM BSA (Stratagene), low salt buffer (20 mM Tris-HCl, pH8.8, 10 mM KCl, 10 mM $(NH_4)_2SO_4$, 2 mM $MgSO_4$ and 0,1% Triton X-100) (Stratagene), 0.25 mM of each deoxynucleoside triphosphate and 0.5 U Taq Plus Long DNA polymerase (Stratagene)). Final volume was 10 µl (all concentrations given are concentrations in the final volume). Predenaturation was carried out at 94°C for 30 s. 30 cycles of the following was performed: Denaturation at 94°C for 30 s, annealing at 55°C for 30 s and elongation at 72°C for 1 min.

The following primer combinations were used (the length of the amplified products are given in parentheses):

*cfp7*: pVF1 and PVR1 (274 bp), pVF1 and PVR2 (197 bp), pVF3 and PVR1 (302 bp), pVF3 and PVR2 (125 bp).

TABLE 10.

Mycobacterial strains used in this Example.

| Species and strain(s) | | Source |
|---|---|---|
| 1. *M. tuberculosis* | H 3 7 R vATCC[a] | |
| | ( A T C C 27294) | |
| 2. | H 3 7 R aATCC | |
| | (ATCC 25177) | |
| 3. | Erdman | Obtained from A. Lazlo, Ottawa, Canada |
| 4. *M. bovis* BCG substrain: Danish 1331 | | SSI[b] |
| 5. | Chinese | SSI[c] |
| 6. | Canadian | SSI[c] |
| 7. | Glaxo | SSI[c] |
| 8. | Russia | SSI[c] |
| 9. | Pasteur | SSI[c] |
| 10. | Japan | WHO[e] |
| 11. *M. bovis* MNC 27 | | SSI[c] |
| 12. *M. africanum* | | Isolated from a Danish patient |
| 13. *M. leprae* (armadillo-derived) | | Obtained from J. M. Colston, London, UK |
| 14. *M. avium* (ATCC 15769) | | ATCC |
| 15. *M. kansasii* (ATCC 12478) | | ATCC |
| 16. *M. marinum* (ATCC 927) | | ATCC |
| 17. *M. scrofulaceum* (ATCC 19275) | | ATCC |
| 18. *M. intercellulare* (ATCC 15985) | | ATCC |
| 19. *M. fortuitum* (ATCC 6841) | | ATCC |
| 20. *M. xenopi* 21. *M. flavescens* | | Isolated from a Danish patient Isolated from a Danish patient |
| 22. *M. szulgai* | | Isolated from a Danish patient |
| 23. *M. terrae* | | SSI[c] |
| 24. *E. coli* | | SSI[d] |
| 25. *S. aureus* | | SSI[d] |

[a] American Type Culture Collection, USA.

[b] Statens Serum Institut, Copenhagen, Denmark.

[c] Our collection Department of Mycobacteriology, Statens Serum Institut, Copenhagen, Denmark.

[d] Department of Clinical Microbiology, Statens Serum Institut, Denmark.

[e] WHO International Laboratory for Biological Standards, Statens Serum Institut, Copenhagen, Denmark.

TABLE 11.

Sequence of the *cfp7* oligonucleotide

| Orientation and oligonucleotide | Sequences (5'-3')[a] | Position[b] (nucleotides) |
|---|---|---|
| Sense | | |
| pvR1 | GTACGAGAATTCATGTCGCAAATCATG (SEQ ID NO: 35) | 91 - 105 (SEQ ID NO: 1) |
| pvR2 | GTACGAGAATTCGAGCTTGGGGTGCCG (SEQ ID NO: 36) | 168-181 (SEQ ID NO: 1) |
| Antisense | | |
| pvF1 | CGTTAGGGATCCTCATCGCCATGGTGTTGG (SEQ ID NO: 38) | 340 - 323 (SEQ ID NO: 1) |

(continued)

Sequence of the *cfp7* oligonucleotide

| Orientation and oligonucleotide | Sequences (5'-3')[a] | Position[b] (nucleotides) |
|---|---|---|
| pvF3 | CGTTAGGGATCCGGTTCCACTGTGCC (SEQ ID NO: 39) | 268 - 255 (SEQ ID NO: 1) |

[a] Nucleotides underlined are not contained in the nucleotide sequence *cfp7*.

[b] The positions referred to are of the non-underlined parts of the primers and correspond to the nucleotide sequence shown in SEQ ID NO: 1 for *cfp7*.

The Southern blotting was carried out as described previously (Oettinger and Andersen, 1994) with the following modifications: 2 μg of genomic DNA was digested with *Pvu*II, electrophoresed in an 0.8% agarose gel, and transferred onto a nylon membrane (Hybond N-plus; Amersham International plc, Little Chalfont, United Kingdom) with a vacuum transfer device (Milliblot, TM-v; Millipore Corp., Bedford, MA). The *cfp7,* gene fragments were amplified by PCR from the plasmid pRVN01 by using the primers shown in TABLE 11. The probes were labelled non-radioactively with an enhanced chemiluminescence kit (ECL; Amersham International plc, Little Chalfont, United Kingdom). Hybridization and detection was performed according to the instructions provided by the manufacturer. The results are summarized in TABLE 12.

TABLE 12. Interspecies analysis of the *cfp7, cfp9* and *mpt51* genes by PCR and/or Southern blotting and of MPT51 protein by Western blotting.

| | | | PCR | Southern blot |
|---|---|---|---|---|
| Species and strain | | | *cfp7* | *cfp7* |
| 1. | | *M. tub.* H37Rv | + | + |
| 2. | | *M. tub.* H37Ra | + | N.D. |
| 3. | | *M. tub.* Erdmann | + | + |
| 4. | | *M. bovis* | + | |
| 5. | | *M. bovis* BCG Danish 1331 | + | + |
| 6. | | *M. bovis* BCG Japan | + | + |
| 7. | | *M. bovis* BCG Chinese | + | + |
| 8. | | *M. bovis* BCG Canadian | + | + |
| 9. | | *M. bovis* BCG Glaxo | + | + |
| 10. | | *M. bovis* BCG Russia | + | + |
| 11. | | *M. bovis* BCG Pasteur | + | + |
| 12. | | M. *africanum* | + | + |
| 13. | | *M. leprae* - | - | - |
| 14. | | M. avium | + | + |
| 15. | | M. *kansasii* | + | + |
| 16. | | M. *marinum* | - | + |
| 17. | | M. *scrofulaceum* | - | - |
| 18. | | M. *intercellulare* | + | + |
| 19. | | *M. fortuitum* | | |
| 20. | | *M. flavescens* | + | + |
| 21. | | *M. xenopi* | - | N.D. |
| 22. | | M. *szulga*i | (+) | - |
| 23. | | *M. terrae* | - | N.D. |

+, positive reaction; -,no reaction, N.D. not determined.

*cfp7* was found in the M. *tuberculosis* complex including BCG and the environmental mycobacteria; *M. avium, M. kansasii, M. marinum, M. intracellular* and *M. flavescens.*

LIST OF REFERENCES

Andersen, P. and Heron, I, 1993, J. Immunol. Methods 161: 29-39.

Andersen, A. B. et al., 1992, Infect. Immun. 60: 2317-2323.

Andersen P., 1994, Infect. Immun. 62: 2536-44.

Andersen P. et al., 1995, J. Immunol. 154: 3359-72

Barkholt, V. and Jensen, A. L., 1989, Anal. Biochem. 177: 318-322.

Borodovsky, M., and J. McIninch. 1993, Computers Chem. 17: 123-133.

van Dyke M. W. et al., 1992. Gene pp. 99-104.

Gosselin et al., 1992, J. Immunol. 149: 3477-3481.

Harboe, M. et al., 1996, Infect. Immun. 64: 16-22.

von Heijne, G., 1984, J. Mol. Biol. 173: 243-251.

Hochstrasser, D.F. et al., 1988, Anal.Biochem. 173: 424-435

Köhler, G. and Milstein, C., 1975, Nature 256: 495-497.

Li, H. et al., 1993, Infect. Immun. 61: 1730-1734.

Lindblad E.B. et al., 1997, Infect. Immun. 65: 623-629.

Mahairas, G. G. et al., 1996, J. Bacteriol 178: 1274-1282.

Maniatis T. et al., 1989, "Molecular cloning: a laboratory manual", 2nd ed., Cold Spring Harbor Laboratory, Cold Spring Harbor, N.Y.

Nagai, S. et al., 1991, Infect. Immun. 59: 372-382.

Oettinger, T. and Andersen, Å. B., 1994, Infect. Immun. 62: 2058-2064.

Ohara, N. et al., 1995, Scand. J. immunol. 41: 233-442.

Pal P. G. and Horwitz M. A., 1992, Infect. Immun. 60: 4781-92.

Pearson, W. R. and Lipman D. J., 1988. Proc. Natl. Acad. Sci. USA 85: 2444-2448.

Ploug, M. et al., 1989, Anal. Biochem. 181: 33-39.

Porath, J. et al., 1985, FEBS Lett. 185: 306-310.

Roberts, A.D. et al., 1995, Immunol. 85: 502-508.

Sørensen, A.L. et al., 1995, Infect. Immun. 63: 1710-1717.

Theisen, M. et al., 1995, Clinical and Diagnostic Laboratory Immunology, 2: 30-34.

Valdés-Stauber, N. and Scherer, S., 1994, Appl. Environ. Microbiol. 60: 3809-3814.

**EP 1 449 922 B1**

Valdés-Stauber, N. and Scherer, S., 1996, Appl. Environ. Microbiol. 62: 1283-1286.

Williams, N., 1996, Science 272: 27.

Young, R. A. et al., 1985, Proc. Natl. Acad. Sci. USA 82: 2583-2587.

**Claims**

1.  A preparation of a polypeptide fragment, which

    a) comprises an amino acid sequence as shown in SEQ ID NO: 2,
    b) comprises a subsequence of the polypeptide fragment defined in a) which has a length of at least 12 amino acid residues, said subsequence being immunologically equivalent to the polypeptide defined in a) with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex, or
    c) comprises an amino acid sequence having a sequence identity with the polypeptide defined in a) or the subsequence defined in b) of at least 80% and at the same time being immunologically equivalent to the polypeptide defined in a) with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex,

    said preparation containing at most 5% by weight of other polypeptide material with which the polypeptide fragment is natively associated, with the proviso that, when consisting of the amino acid sequence 1-96 of SEQ ID NO: 2, the polypeptide fragment is free of any other antigen from bacteria belonging to the tuberculosis complex..

2.  A preparation according to claim 1, wherein said polypeptide fragment comprises an epitope for a T-helper cell.

3.  The preparation according to claim 1 or 2, which

    1) induces a release of IFN-γ from primed memory T-lymphocytes withdrawn from a mouse within 2 weeks of primary infection or within 4 days after the mouse has been re-challenge infected with mycobacteria belonging to the tuberculosis complex, the induction performed by the addition of the polypeptide to a suspension comprising about 200.000 spleen cells per ml, the addition of the polypeptide resulting in a concentration of 1-4 µg polypeptide per ml suspension, the release of IFN-γ being assessable by determination of IFN-γ in supernatant harvested 2 days after the addition of the polypeptide to the suspension, and/or
    2) induces a release of IFN-γ of at least 300 pg above background level from about 1,000,000 human PBMC (peripheral blood mononuclear cells) per ml isolated from TB patients in the first phase of infection, or from healthy BCG vaccinated donors, or from healthy contacts to TB patients, the induction being performed by the addition of the polypeptide to a suspension comprising the about 1,000,000 PBMC per ml, the addition of the polypeptide resulting in a concentration of 1-4 µg polypeptide per ml suspension, the release of IFN-γ being assessable by determination of IFN-γ in supernatant harvested 2 days after the addition of the polypeptide to the suspension; and/or

3) induces an IFN-γ release from bovine PBMC derived from animals previously sensitized with mycobacteria belonging to the tuberculosis complex, said release being at least two times the release observed from bovine PBMC derived from animals not previously sensitized with mycobacteria belonging to the tuberculosis complex.

4. The preparation according to any of the preceding claims, wherein the polypeptide fragment is free of any other antigen from bacteria belonging to the tuberculosis complex.

5. The preparation according to any of the preceding claims, wherein the polypeptide fragment is free from any signal sequence.

6. A preparation according to any of the preceding claims, wherein the sequence identity with the polypeptide shown in SEQ ID NO: 2 or with subsequences of said polypeptide having having at least 12 amino acid residues is at least 85%, at least 90%, at least 91%, at least 92%, at least 93%, at least 94%, at least 95%, at least 96%, at least 97%, at least 98%, at least 99%, and at least 99.5%.

7. A preparation according to any of the preceding claims wherein the polypeptide fragment is lipidated so as to allow a self-adjuvating effect of the polypeptide.

8. A fusion polypeptide comprising at least one polypeptide fragment as defined in any of the preceding claims and at least one fusion partner.

9. A fusion polypeptide according to claim 8, wherein the fusion partner is selected from the group consisting of a polypeptide fragment as defined in any of claims 1-7, and an other polypeptide fragment derived from a bacterium belonging to the tuberculosis complex.

10. A preparation of a polypeptide fragment according to any of claims 1-9 for use as a pharmaceutical, said preparation containing at most 5% by weight of other polypeptide material with which the polypeptide fragment is natively associated.

11. The use of a preparation of a polypeptide fragment according to any of claims 1-9 in the preparation of a pharmaceutical composition for the diagnosis of or vaccination against tuberculosis caused by *Mycobacterium tuberculosis, Mycobacterium africanum* or *Mycobacterium bovis,* said preparation containing at most 5% by weight of other polypeptide material with which the polypeptide fragment is natively associated.

12. A nucleic acid fragment in isolated form which comprises a nucleic acid sequence which encodes a polypeptide as defined in any of claims 1-9, or comprises a nucleic acid sequence complementary thereto.

13. A nucleic acid fragment according to claim 12, which is a DNA fragment.

14. A vaccine comprising a nucleic acid fragment according to claim 12 or 13, the vaccine effecting *in vivo* expression of antigen by an animal, including a human being, to whom the vaccine has been administered, the amount of expressed antigen being effective to confer substantially increased resistance to infections with mycobacteria of the tuberculosis complex in an animal, including a human being.

15. A nucleic acid fragment according to claim 12 or 13 for use as a pharmaceutical.

16. The use of a nucleic acid fragment according to claim 12 or 13 in the preparation of a pharmaceutical composition for the diagnosis of or vaccination against tuberculosis caused by *Mycobacterium tuberculosis, Mycobacterium africanum* or *Mycobacterium bovis.*

17. An immunologic composition comprising a polypeptide fragment as defined in any of claims 1-9.

18. An immunologic composition according to claim 17, which further comprises an immunologically and pharmaceutically acceptable carrier, vehicle or adjuvant.

**19.** An immunologic composition according to claim 18, wherein the carrier is selected from the group consisting of a polymer to which the polypeptide(s) is/are bound by hydrophobic non-covalent interaction, such as a plastic, e.g. polystyrene, a polymer to which the polypeptide(s) is/are covalently bound, such as a polysaccharide, and a polypeptide, e.g. bovine serum albumin, ovalbumin or keyhole limpet hemocyanin; the vehicle is selected from the group consisting of a diluent and a suspending agent; and the adjuvant is selected from the group consisting of dimethyldioctadecylammonium bromide (DDA), Quil A, poly I:C, Freund's incomplete adjuvant, IFN-γ, IL-2, IL-12, monophosphoryl lipid A (MPL), and muramyl dipeptide (MDP).

**20.** An immunologic composition according to any of claims 17 to 19, comprising at least two different polypeptide fragments, each different polypeptide fragment being a polypeptide fragment as defined in any of claims 1-9.

**21.** An immunologic composition according to claim 20, comprising 3-20 different polypeptide fragments, each different polypeptide fragment being as defined in any of claims 1-9.

**22.** An immunologic composition according to any of claims 15-19, which is in the form of a vaccine.

**23.** An immunologic composition according to any of claims 17-21, which is in the form of a skin test reagent.

**24.** A vaccine for immunizing an animal, including a human being, against tuberculosis caused by mycobacteria belonging to the tuberculosis complex, comprising as the effective component a non-pathogenic microorganism, wherein at least one copy of a DNA fragment comprising a DNA sequence encoding a polypeptide fragment as defined in any of claims 1-9 has been incorporated into the genome of the microorganism in a manner allowing the microorganism to express and optionally secrete the polypeptide.

**25.** A vaccine according to claim 24, wherein the microorganism is a bacterium.

**26.** A vaccine according to claim 25, wherein the bacterium is selected from the group consisting of the genera *Mycobacterium, Salmonella, Pseudomonas* and *Eschericia.*

**27.** A vaccine according to claim 26, wherein the microorganism is *Mycobacterium bovis* BCG, such as *Mycobacterium bovis* BCG strain: Danish 1331.

**28.** A vaccine according to any of claims 24-27, wherein at least 2 copies of a DNA fragment encoding a polypeptide according to any of claims 1-9 are incorporated into the genome of the microorganism.

**29.** A vaccine according to claim 28, wherein the number of copies is at least 5.

**30.** A replicable expression vector which comprises a nucleic acid fragment according to claim 12 or 13.

**31.** A vector according to claim 30, which is selected from the group consisting of a virus, a bacteriophage, a plasmid, a cosmid, and a microchromosome.

**32.** A transformed cell harbouring at least one vector according to claim 30 or 31.

**33.** A transformed cell according to claim 32, which is a bacterium belonging to the tuberculosis complex, such as a *M. tuberculosis bovis* BCG cell.

**34.** A transformed cell according to claim 32 or 33, which expresses a polypeptide fragment as defined in any of claims 1-9.

**35.** A method for producing a polypeptide fragment as defined in any of claims 1-9, comprising

inserting a nucleic acid fragment according to claim 12 or 13 into a vector which is able to replicate in a host cell, introducing the resulting recombinant vector into the host cell, culturing the host cell in a culture medium under conditions sufficient to effect expression of the polypeptide, and recovering the polypeptide from the host cell or culture medium; or
isolating the polypeptide from a short-term culture filtrate as defined in claim 1; or
isolating the polypeptide from whole mycobacteria of the tuberculosis complex or from lysates or fractions thereof, e.g. cell wall containing fractions; or

synthesizing the polypeptide by solid or liquid phase peptide synthesis.

36. A method for producing an immunologic composition according to any of claims 17-21 comprising

preparing, synthesizing or isolating a polypeptide fragment as defined in any of claims 1-9, and
solubilizing or dispersing the polypeptide in a medium for a vaccine, and
optionally adding other *M. tuberculosis* antigens and/or a carrier, vehicle and/or adjuvant substance,

or
cultivating a cell according to any of claims 32-34, and
transferring the cells to a medium for a vaccine, and
optionally adding a carrier, vehicle and/or adjuvant substance.

37. An *in vitro* method for diagnosing ongoing or previous sensitization in an animal or a human being with bacteria belonging to the tuberculosis complex, the method comprising contacting a blood sample from the animal with the polypeptide fragment as defined in any of claims 1-9, a significant release into the extracellular phase of at least one cytokine by mononuclear cells in the blood sample being indicative of the animal being sensitized.

38. A composition for diagnosing tuberculosis in an animal, including a human being, comprising a polypeptide fragmentas defined in of claims 1-9, or a nucleic acid fragment according to claim 12 or 13, optionally in combination with a means for detection.

39. A monoclonal or polyclonal antibody, which is specifically reacting with a polypeptide fragment in an immuno assay, or a specific binding fragment of said antibody, wherein said polypeptide fragment is selected from:

a) an amino acid sequence as shown in SEQ ID NO: 2,
b) a subsequence of the polypeptide fragment defined in a) which has a length of at least 12 amino acid residues, said subsequence being immunologically equivalent to the polypeptide defined in a) with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex, or
c) an amino acid sequence having a sequence identity with the polypeptide defined in a) or the subsequence defined in b) of at least 80% and at the same time being immunologically equivalent to the polypeptide defined in a) with respect to the ability of evoking a protective immune response against infections with mycobacteria belonging to the tuberculosis complex or with respect to the ability of eliciting a diagnostically significant immune response indicating previous or ongoing sensitization with antigens derived from mycobacteria belonging to the tuberculosis complex.

**Patentansprüche**

1. Präparat eines Polypeptidfragments, das

a) eine Aminosäuresequenz, wie in SEQ ID NR: 2 gezeigt, umfasst,
b) eine Teilsequenz des in a) definierten Polypeptidfragments umfasst, das eine Länge von mindestens 12 Aminosäureresten hat, wobei die Teilsequenz mit dem in a) definierten Polypeptid immunologisch äquivalent ist hinsichtlich der Fähigkeit, eine schützende immunologische Abwehrreaktion gegen Infektionen mit Mycobacterien, die zum Tuberkulosekomplex gehören, hervorzurufen, oder hinsichtlich der Fähigkeit, eine diagnostische signifikante immunologische Abwehrreaktion auszulösen, die vorhergehende oder anhaltende Sensibilisierung mit Antigenen angibt, die aus Mycobakterien, die zum Tuberkulosekomplex gehören, abgeleitet sind, oder
c) eine Aminosäuresequenz umfasst, die eine Sequenzidentität zu dem in a) definierten Polypeptid oder der in b) definierten Teilsequenz von mindestens 80% hat und gleichzeitig mit dem in a) definierten Polypeptid immunologisch äquivalent ist hinsichtlich der Fähigkeit, eine schützende immunologische Abwehrreaktion gegen Infektionen mit Mycobacterien, die zum Tuberkulosekomplex gehören, hervorzurufen, oder hinsichtlich der Fähigkeit, eine diagnostisch signifikante immunologische Abwehrreaktion auszulösen, die vorhergehende oder anhaltende Sensibilisierung mit Antigenen angibt, die aus Mycobakterien, die zum Tuberkulosekomplex gehören, abgeleitet sind,

wobei das Präparat höchstens 5 Gewichtprozent eines anderen Polypeptidmaterials enthält, mit dem das Polypeptidfragment ursprünglich verknüpft ist, mit der Maßgabe, dass, wenn das Polypeptidfragment aus der Aminosäuresequenz 1-96 mit der SEQ ID Nr.: 2 besteht, das Polypeptidfragment frei von irgendeinem anderen Antigen aus Bakterien, die zum Tuberkulosekomplex gehören, ist.

2. Präparat nach Anspruch 1, worin das Polypeptidfragment ein Epitop für eine T-Hilfszelle umfasst.

3. Präparat nach Anspruch 1 oder 2, das

1) eine Freisetzung von IFN-γ aus geprägten Memory-T-Lymphozyten induziert, die aus einer Maus, innerhalb von 2 Wochen einer ersten Infektion oder innerhalb von 4 Tagen, nachdem die Maus mit Mycobakterien, die zum Tuberkulosekomplex gehören, Re-challengeinfiziert worden war, entnommen worden sind, wobei die Induktion durch den Zusatz des Polypeptids zu einer Suspension, die etwa 200.000 Milzzellen pro ml umfasst, durchgeführt wird, der Zusatz des Polypeptids zu einer Konzentration von 1-4 μg Polypeptid pro ml Suspension führt, die Freizetzung von IFN-γ durch Bestimmung von IFN-γ im Überstand, der 2 Tage nach Zusatz des Polypeptids zur Suspension geerntet wird, bewertbar ist, und/oder
2) eine Freisetzung von IFN-γ von mindestens 300 pg über dem Hintergrundwert von ungefähr 1,000,000 menschlichen PBMC (periferen Blut-Mononuklearzellen) pro ml induziert, die aus TB-Patienten in der ersten Infektionsphase oder aus gesunden, mit BCG geimpften Spendern oder aus gesunden Kontakten mit TB-Patienten isoliert worden sind, wobei die Induktion durch den Zusatz des Polypeptids zu einer Suspension, die etwa 1,000,000 PBMC pro ml umfasst, wobei der Zusatz des Polypeptids zu einer Konzentration von 1-4 μg Polypeptid pro ml Suspension führt, die Freisetzung von IFN-γ durch Bestimmung von IFN-γ im Überstand, der 2 Tage nach dem Zusatz des Polypeptids zur Suspension geerntet wird, bewertbar ist, und/oder
3) eine IFN-γ-Freisetzung von Rinder-PBMC induziert, das aus Tieren, die vorher mit Mycobakterien, die zum Tuberkulosekomplex gehören, sensibilisiert worden sind, wobei die Freisetzung mindestens zweimal die Freisetzung, die bei Rinder-PBMC beobachtet wird, das aus Tieren, die nicht vorher mit Mycobakterien, die zum Tuberkulosekomplex gehören, sensibilsiert worden sind, abgeleitet sind.

4. Präparat nach irgendeinem der vorhergehenden Ansprüche, worin das Polypeptidfragment frei von irgendeinem anderen Antigen aus Bakterien, die zum Tuberkulosekomplex gehören, ist.

5. Präparat nach irgendeinem der vorhergehenden Ansprüche, worin das Polypeptidfragment frei von irgendeiner Signalsequenz ist.

6. Präparat nach irgendeinem der vorhergehenden Ansprüche, worin die Sequenzidentität zu dem Polypeptid, gezeigt in SEQ ID NR: 2 oder zu Teilsequenzen des Polypeptids mit mindestens 12 Aminosäureresten, mindestens 85%, mindestens 90%, mindestens 91%, mindestens 92%, mindestens 93%, mindestens 94%, mindestens 95%, mindestens 96%, mindestens 97%, mindestens 98%, mindestens 99% oder mindestens 99,5% ist.

7. Präparat nach irgendeinem der vorhergehenden Ansprüche, worin das Polypeptidfragment lipidisiert ist, um somit einen selbst-adjuvenierenden Effekt auf das Polypeptid zu genehmigen.

8. Fusionspolypeptid, das mindestens ein Polypeptidfragment, wie in irgendeinem der vorhergehenden Ansprüche definiert, und mindestens einen Fusionspartner umfasst.

9. Fusionspolypeptid nach Anspruch 8, worin der Fusionspartner aus der Gruppe bestehend aus einem Polypeptidfragment, wie in irgendeinem der Ansprüche 1-7 definiert, und ein anderes Polypeptidfragment aus einem Bakterium, das zum Tuberkulosekomplex gehört, abgeleitet ist, gewählt ist.

10. Präparat eines Polypeptidfragments nach irgendeinem der Ansprüche 1-9 zur Verwendung als ein Arzneimittel, wobei das Präparat höchstens 5 Gewichtsprozent eines anderen Polypeptidmaterials enthält, mit dem das Polypeptidfragment ursprünglich verknüpft ist.

11. Verwendung eines Präparats eines Polypeptidfragments nach irgendeinem der Ansprüche 1-9 bei der Herstellung eines pharmazeutischen Zusammensetzung zur Diagnose von oder Impfung gegen Tuberkulose, verursacht durch *Mycobacterium tuberculosis, Mycobacterium africanum* oder *Mycobacterium bovis,* wobei das Präparat höchstens 5 Gewichtprozent eines anderem Polypeptidmaterial enthält, mit der das Polypeptidfragment ursprünglich verknüpft ist.

**12.** Nukleinsäurefragment in isolierter Form, das eine Nukleinsäuresequenz umfasst, das ein Polypeptid, wie in irgendeinem der Ansprüche 1-9 definiert, kodiert oder eine dazu komplementäre Nukleinsäuresequenz umfasst.

**13.** Nukleinsäurefragmet nach Anspruch 12, das ein DNA-Fragment ist.

**14.** Vaccine, umfassend ein Nukleinsäurefragment nach Anspruch 12 oder 13, wobei die Vaccine eine *in vivo*-Expression von Antigenen durch ein Tier, einschliesslich eines Menschen, dem die Vaccine verabreicht worden ist, bewirkt, wobei die Menge an exprimierten Antigenen wirksam ist, um eine im Wesentlichen erhöhte Resistenz gegenüber Infektionen mit Mycobakterien des Tuberkulosekomplexes in einem Tier, einschliesslich eines Menschen, zu verleihen.

**15.** Nukleinsäurefragment nach Anspruch 12 oder 13 zur Verwendung als ein Arzneimittel.

**16.** Verwendung eines Nukleinsäurefragments nach Anspruch 12 oder 13 in der Herstellung einer pharmazeutischen Zusammensetzung für die Diagnose von oder Impfung gegen Tuberkulose, verursacht durch *Mycobacterium tuberculosis, Mycobacterium africanum* oder *Mycobacterium bovis.*

**17.** Immunologische Zusammensetzung, umfassend ein Polypeptidfragment, wie in irgendeinem der Ansprüche 1-9 definiert.

**18.** Immunologische Zusammensetzung nach Anspruch 17, die weiterhin einen immunologisch oder pharmazeutisch verträglichen Träger, ein immunologisch oder pharmazeutisch verträgliches Vehikel oder Adjuvans umfasst.

**19.** Immunologische Zusammensetzung nach Anspruch 18, worin der Träger aus der Gruppe bestehend aus einem Polymer, an das das/die Polypeptid/e durch eine hydrophobe nicht-kovalente Wechselwirkung, wie Plastik, z.B. Polystyrol, gebunden ist/sind, ein Polymer, an das das/die Polypeptid/e kovalent gebunden ist/sind, z.B. Polysaccharide, und ein Polypeptid, z.B. Rinderserum-Albumin, Ovalbumin oder Keyhole Limpet Hemocyanin gewählt ist; das Vehikel aus der Gruppe bestehend aus einem Verdünnungsmittel und einem Stellmittel gewählt ist; und die Adjuvans aus der Gruppe bestehend aus Dimethyldioctadecylammoniumbromid (DDA), Quil A, Poly I:C, Freund's unvollständige Adjuvans, IFN-γ, IL-2, IL-12, Monophosphoryl-Lipid A (MPL) und Muramyl-Dipeptid (MDP) gewählt ist.

**20.** Immunologische Zusammensetzung nach irgendeinem der Ansprüche 17 bis 19, umfassend mindestens zwei verschiedene Polypeptidfragmente, wobei jedes verschiedene Polypeptidfragment ein Polypeptidfragment, wie in irgendeinem der Ansprüche 1-9 definiert, ist.

**21.** Immunologische Zusammensetzung nach Anspruch 20, umfassend 3-20 verschiedene Polypeptidfragmente, wobei jedes verschiedene Polypeptidfragment so ist, wie in irgendeinem der Ansprüche 1-9 definiert.

**22.** Immunologische Zusammensetzung nach irgendeinem der Ansprüche 15-19, das in der Form einer Vaccine ist.

**23.** Immunologische Zusammensetzung nach irgendeinem der Ansprüche 17-21, das in der Form einer Hauttestreagenz ist.

**24.** Vaccine zur Immunisierung eines Tiers, einschliesslich eines Menschen, gegen Tuberkulose, verursacht durch Mycobacterien, die zum Tuberkulosekomplex gehören, das als wirksame Komponente einen nicht-pathogenen Mikroorganismus umfasst, worin mindestens eine Kopie eines DNA-Fragments, das eine DNA-Sequenz umfasst, die für ein Polypeptidfragment, wie in irgendeinem der Ansprüche 1-9 definiert, kodiert, in das Genom des Mikroorganismus derart inkorporiert ist, dass der Mikroorganismus das Polypeptid exprimieren und gegebenenfalls sezernieren kann.

**25.** Vaccine nach Anspruch 24, worin der Mikroorganismus ein Bakterium ist.

**26.** Vaccine nach Anspruch 25, worin das Bakterium aus der Gruppe bestehend aus den Gattungen *Mycobacterium, Salmonella, Pseudomonas* und *Eschericia* gewählt ist.

**27.** Vaccine nach Anspruch 26, worin der Mikroorganismus ein *Mycobacterium bovis* BCG, wie *Mycobacterium bovis* BCG-Stamm: Danish 1331 ist.

**28.** Vaccine nach irgendeinem der Ansprüche 24-27, worin mindestens zwei Kopien eines DNA-Fragments, das für ein Polypeptid nach irgendeinem der Ansprüche 1-9 kodiert, in das Genom des Mikroorganismus inkorporiert ist.

**29.** Vaccine nach Anspruch 28, worin die Kopienzahl mindestens 5 ist.

**30.** Replizierbarer Expressionsvektor, der ein Nukleinsäurefragment nach Anspruch 12 oder 13 umfasst.

**31.** Vektor nach Anspruch 30, der aus der Gruppe, bestehend aus einem Virus, einer Bakteriophage, einem Plasmid, einem Cosmid und einem Mikrochromosom, gewählt ist.

**32.** Transformierte Zelle, die mindestens einen Vektor nach Anspruch 30 oder 31 beherbergt.

**33.** Transformierte Zelle nach Anspruch 32 ein Bakterium ist, das zum Tuberkulosekomplex gehörigt, wie eine *M. Tuberkulose bovis* BCG-Zelle.

**34.** Transformierte Zelle nach Anspruch 32 oder 33, die ein Polypeptidfragment, wie in irgendeinem der Ansprüche 1-9 definiert, ausdrückt.

**35.** Verfahren zur Herstellung eines Polypeptidfragments, wie in irgendeinem der Ansprüche 1-9 definiert, umfassend

Insertieren eines Nukleinsäurefragments nach Anspruch 12 oder 13 in einen Vektor, der fähig ist, in einer Wirtszelle zu replizieren, Einführen des resultierenden rekombinanten Vektors in die Wirtszelle, Kultivieren der Wirtszelle in einem Kulturmedium unter Bedingungen, die geeignet sind, Expression des Polypeptids zu bewirken, und Gewinnen des Polypeptid aus der Wirtszelle oder dem Kulturmedium; oder
Isolieren des Polypeptids aus einem Kurzzeitkulturfiltrat, wie es in Anspruch 1 definiert ist; oder
Isolieren des Polypeptids aus ganzen Mycobacterien des Tuberkulosekomplexes oder aus Lysaten oder Fraktionen davon, z.B. Fraktionen, die Zellwand enthalten; oder
Synthesieren des Polypeptids durch Fest- oder Flüssigphasepeptidsynthese.

**36.** Verfahren zur Herstellung eines immunologisches Präparats nach irgendeinem der Ansprüche 17-21, umfassend

Herstellen, Synthesieren oder Isolieren eines Polypeptidfragments, wie in irgendeinem der Ansprüche 1-9 definiert, und
Solubisieren oder Dispergieren des Polypeptids in einem Medium für eine Vaccine, und
gegebenenfalls Zusetzen eines anderen *M. tuberculosis* Antigens und/oder eines Trägers, Vehikels und/oder einer Adjuvanssubstanz,

oder
Kultivieren einer Zelle nach irgendeinem der Ansprüche 32-34, und
Transferieren der Zellen in ein Medium für eine Vaccine, und
gegebenenfalls Zusetzen eines Trägers, Vehikels und/oder einer Adjuvanssubstanz.

**37.** *In vitro*-Verfahren zur Diagnostizierung einer anhaltenden oder vorhergehenden Sensibiliserung in einem Tier oder einem Menschen mit Bakterien, die zum Tuberkulosekomplex gehören, wobei das Verfahren umfasst, Kontraktieren einer Blutprobe aus dem Tier mit dem Polypeptidfragment, wie in irgendeinem der Ansprüche 1-9 definiert, eine signifikante Freisetzung von mindestens einem Zytokin in die extrazelluläre Phase durch mononukleare Zellen in der Blutprobe, als indikativ für das Tier sensibilisiert wird.

**38.** Zusammensetzung zur Diagnostizierung von Tuberkulose bei einem Tier, einschliesslich eines Menschen, umfassend ein Polypeptidfragment, wie in den Ansprüchen 1-9 definiert, oder ein Nukleinsäurefragment nach Anspruch 12 oder 13, gegebenenfalls in Kombination mit einem Mittel zur Detektion.

**39.** Monoklonaler oder polyklonaler Antikörper, der spezifisch mit einem Polypeptidfragment in einem Immunoassay reagiert, oder ein spezifisches Bindungsfragment des Antikörpers, worin das Polypeptidfragment ausgewählt ist aus:

a) einer Aminosäuresequenz, wie in SEQ ID NR: 2 gezeigt,
b) einer Teilsequenz des in a) definierten Polypeptidfragments, das eine Länge von mindestens 12 Aminosäureresten hat, wobei die Teilsequenz mit dem in a) definierten Polypeptid immunologisch äquivalent ist hinsichtlich der Fähigkeit, eine schützende immunologische Abwehrreaktion gegen Infektionen mit Mycobacterien, die zum Tuberkulosekomplex gehören, hervorzurufen, oder hinsichtlich der Fähigkeit, eine diagnostische signifikante immunologische Abwehrreaktion auszulösen, die vorhergehende oder anhaltende Sensibilisierung mit Antigenen angibt, die aus Mycobakterien, die zum Tuberkulosekomplex gehören, abgeleitet sind, oder

c) einer Aminosäuresequenz, die eine Sequenzidentität zu dem in a) definierten Polypeptid oder der in b) definierten Teilsequenz von mindestens 80% hat und gleichzeitig mit dem in a) definierten Polypeptid immunologisch äquivalent ist hinsichtlich der Fähigkeit, eine schützende immunologische Abwehrreaktion gegen Infektionen mit Mycobacterien, die zum Tuberkulosekomplex gehören, hervorzurufen, oder hinsichtlich der Fähigkeit, eine diagnostisch signifikante immunologische Abwehrreaktion auszulösen, die vorhergehende oder anhaltende Sensibilisierung mit Antigenen angibt, die aus Mycobakterien, die zum Tuberkulosekomplex gehören, abgeleitet sind.

## Revendications

1. Préparation d'un fragment polypeptidique, qui

a) comprend une séquence d'acide aminé telle que représentée dans SEQ ID NO : 2,

b) comprend une sous-séquence du fragment polypeptidique défini en a) ayant une longueur d'au moins 12 résidus d'acides aminés, ladite sous-séquence étant immunologiquement équivalente au polypeptide défini en a) en ce qui concerne l'aptitude à susciter une réponse immunitaire protectrice contre les infections aux mycobactéries appartenant au complexe tuberculosis ou bien en ce qui concerne l'aptitude à provoquer une réponse immunitaire diagnostiquement significative indiquant une sensibilisation antérieure ou en cours à des antigènes dérivés de mycobactéries appartenant au complexe tuberculosis, ou bien

c) comprend une séquence d'acide aminé ayant une identité de séquence avec le polypeptidique défini en a) ou la sous-séquence définie en b) d'au moins 80 % et étant en même temps immunologiquement équivalente au polypeptide défini en a) en ce qui concerne l'aptitude à susciter une réponse immunitaire protectrice contre les infections aux mycobactéries appartenant au complexe tuberculosis ou bien en ce qui concerne l'aptitude à provoquer une réponse immunitaire diagnostiquement significative indiquant une sensibilisation antérieure ou en cours à des antigènes dérivés de mycobactéries appartenant au complexe tuberculosis,

ladite préparation contenant au plus 5 % en poids d'autre matériau polypeptidique avec lequel est nativement associé le fragment polypeptidique, étant entendu que le fragment polypeptidique, lorsqu'il consiste en la séquence d'acide aminé 1-96 de la SEQ ID NO : 2, est dépourvu de tout autre antigène de bactéries appartenant au complexe tuberculosis.

2. Préparation selon la revendication 1, dans laquelle ledit fragment polypeptidique comprend un épitope d'une cellule T-auxiliaire.

3. Préparation selon la revendication 1 ou 2, qui

1) induit une libération d'IFN-$\gamma$ par des lymphocytes-T à mémoire amorcés soustraits à une souris dans les 2 semaines après la primo-infection ou dans les 4 jours après que la souris ait été ré-exposée à une infection aux mycobactéries appartenant au complexe tuberculosis, l'induction étant effectuée par addition du polypeptide à une suspension comprenant environ 200 000 cellules de rate par ml, l'addition du polypeptide donnant lieu à une concentration de 1-4 $\mu$g de polypeptide par ml de suspension, la libération d'IFN-$\gamma$ pouvant être évaluée par détermination de l'IFN-$\gamma$ dans le surnageant recueilli 2 jours après l'addition du polypeptide à la suspension, et/ou

2) induit une libération d'IFN-$\gamma$ d'au moins 300 au-dessus du niveau de fond par environ 1 000 000 PBMC humains (cellules mononucléaires du sang périphérique) par ml isolés à partir de patients tuberculeux dans la première phase d'infection ou à partir de donneurs vaccinés au BCG ou à partir de contacts sains de patients tuberculeux, l'induction étant effectuée par addition du polypeptide à une suspension comprenant environ 1 000 000 PBMC par ml, l'addition du polypeptide donnant lieu à une concentration de 1-4 $\mu$g de polypeptide par ml de suspension, la libération d'IFN-$\gamma$ pouvant être évaluée par détermination de l'IFN-$\gamma$ dans le surnageant recueilli 2 jours après l'addition du polypeptide à la suspension, et/ou

3) induit une libération d'IFN-$\gamma$ à partir de PBMC bovins dérivés d'animaux préalablement sensibilisés par des mycobactéries appartenant au complexe tuberculosis, ladite libération étant au moins deux fois la libération observée pour des PBMC dérivés d'animaux non préalablement sensibilisés par des mycobactéries appartenant au complexe tuberculosis.

**4.** Préparation selon l'une des revendications précédentes, dans laquelle le fragment polypeptidique est dépourvu de tout autre antigène de bactéries appartenant au complexe tuberculosis.

**5.** Préparation selon l'une des revendications précédentes, dans laquelle le fragment polypeptidique est dépourvu de toute séquence signal.

**6.** Préparation selon l'une des revendications précédentes, dans laquelle l'identité de séquence avec le fragment polypeptidique représenté dans SEQ ID NO : 2 ou avec des sous-séquences dudit polypeptide ayant au moins 12 résidus d'acides aminés est d'au moins 85 %, d'au moins 90 % , d'au moins 91 % , d'au moins 92 %, d'au moins 93 %, d'au moins 94 %, d'au moins 95 %, d'au moins 96 %, d'au moins 97 %, d'au moins 98 %, d'au moins 99 % et d'au moins 99,5 %.

**7.** Préparation selon l'une des revendications précédentes, dans laquelle le fragment polypeptidique est lipidé pour permettre un effet autoadjuvant du polypeptide.

**8.** Polypeptidique de fusion comprenant au moins un fragment polypeptidique tel que défini dans l'une des revendications précédentes et au moins un partenaire de fusion.

**9.** Polypeptide de fusion selon la revendication 8, dans lequel le partenaire de fusion est choisi dans le groupe constitué d'un fragment polypeptidique tel que défini dans l'une des revendications 1 à 7 et d'un autre fragment polypeptidique dérivé d'une bactérie appartenant au complexe tuberculosis.

**10.** Préparation d'un fragment polypeptidique selon l'une des revendications 1 à 9 destiné à une utilisation en tant que médicament, ladite préparation contenant au plus 5 % en poids d'un autre matériau polypeptidique avec lequel est nativement associé le fragment polypeptidique.

**11.** Utilisation d'une préparation d'un fragment polypeptidique selon l'une des revendications 1 à 9 pour la préparation d'une composition pharmaceutique destinée au diagnostic de ou à la vaccination contre la tuberculose causée par *Mycobacterium tuberculosis, Mycobacterium africanum* ou *Mycobacterium bovis,* ladite préparation contenant au plus 5 % en poids d'un autre matériau polypeptidique avec lequel est nativement associé le fragment polypeptidique.

**12.** Fragment d'acide nucléique sous forme isolée qui comprend une séquence d'acide nucléique codant pour un polypeptide tel que défini dans l'une des revendications 1 à 9, ou qui comprend une séquence d'acide nucléique complémentaire à celle-ci.

**13.** Fragment d'acide nucléique selon la revendication 12, qui est un fragment d'ADN.

**14.** Vaccin comprenant un fragment d'acide nucléique selon la revendication 12 ou 13, le vaccin entraînant une expression *in vivo* d'antigène par un animal, y compris un être humain, auquel le vaccin a été administré, la quantité d'antigène exprimé étant efficace pour conférer une résistance substantiellement améliorée aux infections aux mycobactéries du complexe tuberculosis chez un animal, y compris un être humain.

**15.** Fragment d'acide nucléique selon la revendication 12 ou 13 destiné à une utilisation en tant que médicament.

**16.** Utilisation d'un fragment d'acide nucléique selon la revendication 12 ou 13 dans la préparation d'une composition pharmaceutique destinée au diagnostic de ou à la vaccination contre la tuberculose causée par *Mycobacterium tuberculosis, Mycobacterium africanum* ou *Mycobacterium bovis.*

**17.** Composition immunologique comprenant un fragment polypeptidique tel que défini dans l'une des revendications 1 à 9.

**18.** Composition immunologique selon la revendication 17, comprenant en outre un support, un véhicule ou un adjuvant immunologiquement et pharmaceutiquement acceptables.

**19.** Composition immunologique selon la revendication 18, dans laquelle le support est choisi dans le groupe constitué d'un polymère auquel le(s) polypeptide(s) est/sont lié(s) par interaction hydrophobe non-covalente, tel qu'une matière plastique, par exemple du polystyrène, d'un polymère auquel le(s) polypeptide(s) est/sont lié(s) de façon covalente, tel qu'un polysaccharide et un polypeptide, par exemple le sérumalbumine bovin, l'ovalbumine ou l'hémocyanine de patelle (Keyhole Limpet) ; le véhicule est choisi dans le groupe constitué d'un diluant et d'un agent de suspension ; et l'adjuvant est choisi dans le groupe constitué du bromure de diméthyle dioctadécyle ammonium (DDA), du Quil A, du poly I:C, de l'adjuvant incomplet de Freund, de l'IFN-γ, de l'IL-2, de l'IL-12, du monophosphoryle lipide A (MPL) et du muramyl dipeptide (MDP).

**20.** Composition immunologique selon l'une des revendications 17 à 19, comprenant au moins deux fragments polypeptidiques différents, chaque fragment polypeptidique différent étant un fragment polypeptidique tel que défini dans l'une des revendications 1 à 9.

**21.** Composition immunologique selon la revendication 20, comprenant 3 à 20 fragments polypeptidiques différents, chaque fragment polypeptidique différent étant tel que défini dans l'une des revendications 1 à 9.

**22.** Composition immunologique selon l'une des revendications 15 à 19, qui est sous forme d'un vaccin.

**23.** Composition immunologique selon l'une des revendications 17 à 21, qui est sous forme d'un réactif de test cutané.

**24.** Vaccin destiné à immuniser un animal, y compris un être humain, contre la tuberculose causée par des mycobactéries appartenant au complexe tuberculosis, comprenant en tant que composant effectif un microorganisme non-pathogène, dans lequel au moins une copie d'un fragment d'ADN comprenant une séquence d'ADN codant pour un fragment polypept;dique tel que défini dans l'une des revendications 1 à 9 a été incorporé dans le génome du microorganisme de façon à permettre au microorganisme d'exprimer et optionnellement de sécréter polypeptide.

**25.** Vaccin selon la revendication 24, dans lequel le microorganisme est une bactérie.

**26.** Vaccin selon la revendication 25, dans lequel la bactérie est choisie dans le groupe constitué des genres *Mycobacterium, Salmonella, Pseudomonas* et *Eschericia.*

**27.** Vaccin selon la revendication 26, dans lequel le microorganisme est *Mycobacterium bovis* BCG, tel que *Mycobacterium bovis* BCG souche: Danish 1331.

**28.** Vaccin selon l'une des revendications 24 à 27, dans lequel au moins 2 copies d'un fragment d'ADN codant pour un polypeptide selon l'une des revendications 1 à 9 sont incorporées dans le génome du microorganisme.

**29.** Vaccin selon la revendication 28, dans lequel le nombre de copies est d'au moins 5.

**30.** Vecteur d'expression réplicable qui comprend un fragment d'acide nucléique selon la revendication 12 ou 13.

**31.** Vecteur selon la revendication 30, qui est choisi dans le groupe constitué d'un virus, d'un bactériophage, d'un plasmide, d'un cosmide et d'un microchromosome.

**32.** Cellule transformée contenant au moins un vecteur selon la revendication 30 ou 31.

**33.** Cellule transformée selon la revendication 32, qui est une bactérie appartenant au complexe tuberculosis, telle qu'une cellule de *M. tuberculosis bovis* BCG.

**34.** Cellule transformée selon la revendication 32 ou 33, qui exprime un fragment polypeptidique tel que défini dans l'une des revendications 1 à 9.

**35.** Procédé pour produire un fragment polypeptidique tel que défini dans l'une des revendications 1 à 9, comprenant

l'insertion d'un fragment d'acide nucléique selon la revendication 12 ou 13 dans un vecteur capable de se répliquer dans une cellule hôte, l'introduction du vecteur recombinant résultant dans la cellule hôte, la culture de la cellule hôte dans un milieu de culture dans des conditions suffisantes pour entraîner l'expression du polypeptide, et la récupération du polypeptide à partir de la cellule hôte ou du milieu de culture ; ou bien l'isolation du polypeptide à partir d'un filtrat de culture à court terme tel que défini dans la revendication 1 ; ou bien l'isolation du polypeptide à partir de mycobactéries entières du complexe de tuberculose ou à partir de lysats ou de fractions de celles-ci, par exemple des fractions contenant des parois cellulaires ; ou
la synthèse du polypeptide par synthèse peptidique en phase solide ou liquide.

**36.** Procédé pour produire une composition immunologique selon l'une des revendications 17 à 21, comprenant

la préparation, la synthèse ou l'isolation d'un fragment polypeptidique tel que défini dans l'une des revendications 1 à 9, et
la solubilisation ou la dispersion du polypeptide dans un milieu pour un vaccin, et
optionnellement l'addition d'autres antigènes de *M. tuberculosis* et/ou d'un support, d'un véhicule et/ou d'une substance adjuvante,

ou
la culture d'une cellule selon l'une des revendications 32 à 34, et
le transfert des cellules dans un milieu pour un vaccin, et
optionnellement l'addition d'un support, d'un véhicule et/ou d'une substance adjuvante.

**37.** Procédé pour le diagnostic *in vitro* d'une sensibilisation en cours ou antérieure d'un animal ou d'un être humain par des bactéries appartenant au complexe tuberculosis, le procédé comprenant la mise en contact d'un échantillon de sang de l'animal avec le fragment polypeptidique tel que défini dans l'une des revendications 1 à 9, une libération significative par les cellules mononucléaires de l'échantillon de sang d'au moins une cytokine dans la phase extracellulaire indiquant que l'animal est sensibilisé.

**38.** Composition destinée au diagnostic de la tuberculose chez un animal, y compris un être humain, comprenant un fragment polypeptidique tel que défini dans l'une des revendications 1 à 9 ou un fragment d'acide nucléique selon la revendication 12 ou 13, optionnellement en combinaison avec un moyen de détection.

**39.** Anticorps monoclonal ou polyclonal, qui réagit de façon spécifique avec un fragment polypeptidique dans un dosage immunologique, ou bien un fragment à liaison spécifique dudit anticorps, dans lequel ledit fragment polypeptidique est choisi parmi :

a) une séquence d'acide aminé telle que représentée dans SEQ ID NO : 2,
b) une sous-séquence du fragment polypeptidique défini en a) ayant une longueur d'au moins 12 résidus d'acides aminés, ladite sous-séquence étant immunologiquement équivalente au polypeptide défini en a) en ce qui concerne l'aptitude à susciter une réponse immunitaire protectrice contre les infections aux mycobactéries appartenant au complexe tuberculosis ou bien en ce qui concerne l'aptitude à provoquer une réponse immunitaire diagnostiquement significative indiquant une sensibilisation antérieure ou en cours à des antigènes dérivés de mycobactéries appartenant au complexe tuberculosis, ou bien
c) une séquence d'acide aminé ayant une identité de séquence avec le polypeptidique défini en a) ou la sous-séquence définie en b) d'au moins 80 % et étant en même temps immunologiquement équivalente au polypeptide défini en a) en ce qui concerne l'aptitude à susciter une réponse immunitaire protectrice contre les infections aux mycobactéries appartenant au complexe tuberculosis ou bien en ce qui concerne l'aptitude à provoquer une réponse immunitaire diagnostiquement significative indiquant une sensibilisation antérieure ou en cours à des antigènes dérivés de mycobactéries appartenant au complexe tuberculosis.

**Fig. 1**

**Fig. 2**

```
  1   GGCCGCCGGT ACCTATGTGG CCGCCGATGC TGCGGNCGCG TCGACCTATA CCGGGTTCTG     60
                    -35 region                                -10 region

 61   ATCGAACCCT GCTGACCGAG AGGACTTGTG ATG TCG CAA ATC ATG TAC AAC TAC CCC GCG   120
                     Shine Delgarno  M   S   Q   I   M   Y   N   Y   P   A

121   ATG TTG GGT CAC GCC GGG GAT ATG GCC GGA TAT GCC GGC ACG CTG CAG AGC TTG GGT GCC   180
       M   L   G   H   A   G   D   M   A   G   Y   A   G   T   L   Q   S   L   G   A

181   GAG ATC GCC GTG GAG CAG GCC GCG TTG CAG AGT GCG TGG CAG GGC GAT ACC GGG ATC ACG   240
       E   I   A   V   E   Q   A   A   L   Q   S   A   W   Q   G   D   T   G   I   T

241   TAT CAG GCG TGG CAG GCA CAG TGG AAC CAG GCC ATG GAA GAT TTG GTG CGG GCC TAT CAT   300
       Y   Q   A   W   Q   A   Q   W   N   Q   A   M   E   D   L   V   R   Y   H   A

301   GCG ATG TCC AGC ACC CAT GAA GCC AAC ACC ATG GCG ATG ATG GCC CGC GAC ACC GCC GAA   360
       Y   M   S   S   T   H   E   A   N   T   M   A   M   M · A   R   D   T   A   E

361   GCC GCC AAA TGG GGC GGC TAG                                            381
       A   A   K   W   G   G   *
```

# Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0706571 A
- EP 0432203 A
- WO 97709428 A
- WO 9709429 A
- US 4554101 A
- WO 9418227 A
- US 4603102 A
- US 4608251 A
- US 4601903 A
- US 4599231 A
- US 4599230 A
- US 4596792 A
- US 4578770 A
- US 3791932 A
- US 4174384 A
- US 3949064 A
- WO 9501441 A
- EP 0282242 A

**Non-patent literature cited in the description**

- **NIELSEN P E et al.** *Science,* 1991, vol. 254, 1497-1500
- **ANDERSEN et al.** *J. Immunol.,* vol. 161, 29-39
- **ANDERSEN, P. ; HERON, I.** *J. Immunol. Methods,* 1993, vol. 161, 29-39
- **ANDERSEN, A. B. et al.** *Infect. Immun.,* 1992, vol. 60, 2317-2323
- **ANDERSEN P.** *Infect. Immun.,* 1994, vol. 62, 2536-44
- **ANDERSEN P. et al.** *J. Immunol.,* 1995, vol. 154, 3359-72
- **BARKHOLT, V. ; JENSEN, A. L.** *Anal. Biochem.,* 1989, vol. 177, 318-322
- **BORODOVSKY, M. ; J. MCININCH.** *Computers Chem.,* 1993, vol. 17, 123-133
- **VAN DYKE M. W. et al.** *Gene,* 1992, 99-104
- **GOSSELIN et al.** *J. Immunol.,* 1992, vol. 149, 3477-3481
- **HARBOE, M. et al.** *Infect. Immun.,* 1996, vol. 64, 16-22
- **VON HEIJNE, G.** *J. Mol. Biol.,* 1984, vol. 173, 243-251
- **HOCHSTRASSER, D.F. et al.** *Anal.Biochem.,* 1988, vol. 173, 424-435
- **KÖHLER, G. ; MILSTEIN, C.** *Nature,* 1975, vol. 256, 495-497
- **LI, H. et al.** *Infect. Immun.,* 1993, vol. 61, 1730-1734
- **LINDBLAD E.B. et al.** *Infect. Immun.,* 1997, vol. 65, 623-629
- **MAHAIRAS, G. G. et al.** *J. Bacteriol,* 1996, vol. 178, 1274-1282
- Molecular cloning: a laboratory manual. **MANIATIS T. et al.** Cold Spring Harbor Laboratory. Cold Spring Harbor, 1989
- **NAGAI, S. et al.** *Infect. Immun.,* 1991, vol. 59, 372-382
- **OETTINGER, T. ; ANDERSEN, Å. B.** *Infect. Immun.,* 1994, vol. 62, 2058-2064
- **OHARA, N. et al.** *Scand. J. immunol.,* 1995, vol. 41, 233-442
- **PAL P. G. ; HORWITZ M. A.** *Infect. Immun.,* 1992, vol. 60, 4781-92
- **PEARSON, W. R. ; LIPMAN D. J.** *Proc. Natl. Acad. Sci.,* 1988, vol. 85, 2444-2448
- **PLOUG, M. et al.** *Anal. Biochem.,* 1989, vol. 181, 33-39
- **PORATH, J. et al.** *FEBS Lett.,* 1985, vol. 185, 306-310
- **ROBERTS, A.D. et al.** *Immunol.,* 1995, vol. 85, 502-508
- **SØRENSEN, A.L. et al.** *Infect. Immun.,* 1995, vol. 63, 1710-1717
- **THEISEN, M. et al.** *Clinical and Diagnostic Laboratory Immunology,* 1995, vol. 2, 30-34
- **VALDÉS-STAUBER, N. ; SCHERER, S.** *Appl. Environ. Microbiol.,* 1994, vol. 60, 3809-3814
- **VALDÉS-STAUBER, N. ; SCHERER, S.** *Appl. Environ. Microbiol.,* 1996, vol. 62, 1283-1286
- **WILLIAMS, N.** *Science,* 1996, vol. 272, 27
- **YOUNG, R. A. et al.** *Proc. Natl. Acad. Sci.,* 1985, vol. 82, 2583-2587

SEQUENCE LISTING

(1) GENERAL INFORMATION:

    (i) APPLICANT:

        (A) NAME: Statens Seruminstitut
        (B) STREET: Artillerivej 5
        (C) CITY: Copenhagen
        (E) COUNTRY : Denmark
        (F) POSTAL CODE (ZIP): 2300 S

    (ii) TITLE OF INVENTION: Nucleic acid fragments and polypeptide fragments derived from M. tuberculosis

    (iii) NUMBER OF SEQUENCES : 173

    (iv) COMPUTER READABLE FORM:

        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30 (EPO)

(2) INFORMATION FOR SEQ ID NO: 1:

    (i) SEQUENCE CHARACTERISTICS:

        (A) LENGTH: 381 base pairs
        (B) TYPE: nucleic acid
        (C) STRANDEDNESS: double
        (D) TOPOLOGY: circular

    (ii) MOLECULE TYPE: DNA (genomic)

    (vi) ORIGINAL SOURCE:

        (A) ORGANISM: Mycobacterium tuberculosis
        (B) STRAIN: H37Rv

    (ix) FEATURE:

        (A) NAME/KEY: CDS
        (B) LOCATION: 91..381

    (ix) FEATURE:

        (A) NAME/KEY: -35_signal
        (B) LOCATION: 14..19

    (ix) FEATURE:

(A) NAME/KEY: -10_signal
(B) LOCATION: 47..50

(ix) FEATURE:

(A) NAME/KEY: RBS
(B) LOCATION: 78..84

(ix) FEATURE:

(A) NAME/KEY: mat_peptide
(B) LOCATION: 91..381

(xi) SEQUENCE DESCRIPTION: SEQ ID NO: 1:

```
GGCCGCCGGT ACCTATGTGG CCGCCGATGC TGCGGACGCG TCGACCTATA CCGGGTTCTG          60

ATCGAACCCT GCTGACCGAG AGGACTTGTG ATG TCG CAA ATC ATG TAC AAC TAC         114
                                 Met Ser Gln Ile Met Tyr Asn Tyr
                                   1               5

CCC GCG ATG TTG GGT CAC GCC GGG GAT ATG GCC GGA TAT GCC GGC ACG         162
Pro Ala Met Leu Gly His Ala Gly Asp Met Ala Gly Tyr Ala Gly Thr
        10              15                  20

CTG CAG AGC TTG GGT GCC GAG ATC GCC GTG GAG CAG GCC GCG TTG CAG         210
Leu Gln Ser Leu Gly Ala Glu Ile Ala Val Glu Gln Ala Ala Leu Gln
 25                  30                  35                  40

AGT GCG TGG CAG GGC GAT ACC GGG ATC ACG TAT CAG GCG TGG CAG GCA         258
Ser Ala Trp Gln Gly Asp Thr Gly Ile Thr Tyr Gln Ala Trp Gln Ala
            45                  50                  55

CAG TGG AAC CAG GCC ATG GAA GAT TTG GTG CGG GCC TAT CAT GCG ATG         306
Gln Trp Asn Gln Ala Met Glu Asp Leu Val Arg Ala Tyr His Ala Met
            60                  65                  70

TCC AGC ACC CAT GAA GCC AAC ACC ATG GCG ATG ATG GCC CGC GAC ACC         354
Ser Ser Thr His Glu Ala Asn Thr Met Ala Met Met Ala Arg Asp Thr
            75                  80                  85

GCC GAA GCC GCC AAA TGG GGC GGC TAG                                      381
Ala Glu Ala Ala Lys Trp Gly Gly
        90              95
```

(2) INFORMATION FOR SEQ ID NO: 2:

(i) SEQUENCE CHARACTERISTICS:

(A) LENGTH: 96 amino acids
(B) TYPE: amino acid
(D) TOPOLOGY: linear

(ii) MOLECULE TYPE: protein
(xi) SEQUENCE DESCRIPTION: SEQ ID NO: 2:

```
        Met Ser Gln Ile Met Tyr Asn Tyr Pro Ala Met Leu Gly His Ala Gly
         1               5               10              15

        Asp Met Ala Gly Tyr Ala Gly Thr Leu Gln Ser Leu Gly Ala Glu Ile
                     20              25              30

        Ala Val Glu Gln Ala Ala Leu Gln Ser Ala Trp Gln Gly Asp Thr Gly
                 35              40              45

        Ile Thr Tyr Gln Ala Trp Gln Ala Gln Trp Asn Gln Ala Met Glu Asp
             50              55              60

        Leu Val Arg Ala Tyr His Ala Met Ser Ser Thr His Glu Ala Asn Thr
         65              70              75              80



        Met Ala Met Met Ala Arg Asp Thr Ala Glu Ala Ala Lys Trp Gly Gly
                     85              90              95
```